# EUROPEAN PATENT SPECIFICATION

(11) **EP 0 759 472 B1**
(45) Date of publication and mention of the grant of the patent: **12.06.2002**
(21) Application number: 96905024.4
(22) Date of filing: 07.03.1996
(51) Int. Cl.: C12N 15/53, C12N 9/06, C12N 1/19, C12P 13/24, C12N 9/02

(54) **PROCESS FOR PRODUCING TRANS-4-HYDROXY-L-PROLINE**
VERFAHREN ZUR HERSTELLUNG VON TRANS-4-HYDROXY-L-PROLIN
PROCEDE DE PRODUCTION DE TRANS-4-HYDROXY-L-PROLINE

(30) Priority: 07.03.1995 JP 4698895
(43) Date of publication of application: 26.02.1997
(73) Proprietor: KYOWA HAKKO KOGYO CO., Ltd., Chiyoda-ku, Tokyo 100 (JP)
(72) Inventor: OZAKI, Akio, Machida-shi, Tokyo 194 (JP); MORI, Hideo, Machida-shi, Tokyo 194 (JP); SHIBASAKI, Takeshi, Kawasaki-shi, Kanagawa 215 (JP)
(74) Representative: Vossius, Volker, Dr.
(86) International application number: JP9600559
(87) International publication number: WO9627669

(56) References cited:
- EP-A- 0 547 898
- EP-A- 0 555 475
- EP-A- 0 641 862
- JP-A- 7 126 297
- TETRAHEDRON LETT., Vol. 35, No. 26, (1994), BALDWIN J.E. et al., "Substrate Specificity of Proline 4-Hydroxylase: Chemical and Enzymatic Synthesis of 2S,3R,4S-Epoxyproline", p. 4649-4652.
- TETRAHEDRON LETT., Vol. 34, No. 46, (1993), BALDWIN J.E. et al., "Proline 4-Hydroxylase: Stereochemical Course of the Reaction", p. 7489-7492.

## Description

### Field of the Invention

The present invention relates to industrial methods for producing trans-4-hydroxy-L-proline which is useful as a starting compound for medicines and an additive to foods, genes which code for a protein having the enzymatic activity of hydroxylating the 4-position of L-proline and which are useful for the above-mentioned process (hereinafter referred to as L-proline 4-hydroxylase genes), transformants containing the gene, and methods for producing L-proline 4-hydroxylases using the transformants.

### Background of the Invention

The following processes are known as a method for producing trans-4-hydroxy-L-proline using microorganisms.
1) A process in which trans-4-hydroxy-L-proline is produced from 4-hydroxy-2-oxoglutaric acid using microorganisms of the genus Escherichia (Japanese Published Unexamined Patent Application No. 266,995/91)
2) A process in which trans-4-hydroxy-L-proline is produced directly through fermentation using bacteria or fungi (European (EP 0 547 898 A2, and Japanese Published Unexamined Patent Application Nos. 236,980/93 and 245,782/94)
3) A process in which trans-4-hydroxy-L-proline is produced from L-proline using microorganisms of the genus Streptomyces [J. Biol. Chem., 254, 6684 (1979), Biochem. Biophys. Res. Comm., 120, 45, (1984), Tetrahedron Letters, 34, 7489 (1993), and Tetrahedron Letters, 35, 4649 (1994)].

The conventional processes can, however, hardly be performed on an industrial scale for the following reasons:
1) A substrate for producing trans-4-hydroxy-L-proline, such as 4-hydroxy-2-oxoglutaric acid is too expensive and is difficult to obtain.
2) The productivity of trans-4-hydroxy-L-proline is low.
3) The activity of the enzymes that relate to the production of trans-4-hydroxy-L-proline is quite weak.

With respect to the enzyme that catalyzes the production of trans-4-hydroxy-L-proline, it was reported in a paper that L-proline-4-hydroxylase is purified from a microorganism of the genus Streptomyces. However, a method for obtaining the enzyme and physicochemical properties of the enzyme are not described therein. Further, no paper reported that a gene encoding L-proline-4-hydroxylase having the activity of converting free L-proline into trans-4-hydroxy-L-proline in the presence of 2-ketoglutaric acid. and a divalent iron ion had been cloned.

A process in which trans-4-hydroxy-L-proline is produced industrially advantageously using L-proline-4-hydroxylase having a high level of activity has been in demand.

The object of the present invention is to provide methods for efficiently producing trans-4-hydroxy-L-proline from inexpensive and easily-available L-proline by using L-proline 4-hydroxylases, for which, in order to more industrially advantageously produce trans-4-hydroxy-L-proline, L-proline 4-hydroxylase genes and transformants containing the gene are provided. L-proline 4-hydroxylases are produced in large quantities by using the genes and the transformants, and trans-4-hydroxy-L-proline is industrially produced at low costs by using the transformants or the hydroxylases.

### DISCLOSURE OF THE INVENTION

The present invention relates to novel, microorganism-derived L-proline 4-hydroxylase genes, to recombinant DNAs containing the gene, to transformants containing the recombinant DNA, to methods for producing L-proline 4-hydroxylases using the transformants, to the hydroxylases, and to methods for producing trans-4-hydroxy-L-proline using the transformants or the hydroxylases.

The present invention is described in detail hereinunder.

The L-proline-4-hydroxylases of the present invention are enzymes by which free L-proline is hydroxylated in the presence of 2-ketoglutaric acid and a divalent ion to form trans-4-hydroxy-L-proline.

The present invention encompasses any and every protein having the enzymatic activity of hydroxylating the 4-position of L-proline, which includes, for example, a protein having the amino acid sequence indicated by Sequence No. 1, a fused protein having an amino acid sequence that results from the protein or a protein having a partial amino acid sequence of the protein as bonded to a peptide having a partial amino acid sequence of an Escherichia coli-derived β-galactosidase protein, a fused protein having an amino acid sequence that results from the protein having the amino acid sequence indicated by Sequence No. 1 or a protein having a partial amino acid sequence of the protein as bonded to a peptide having a partial amino acid sequence of an E. coli-derived maltose-binded protein, etc. Examples of the fused proteins include a protein having the amino acid sequence as indicated by Sequence No. 18 or 19, etc.

The protein having the amino acid sequence indicated by Sequence No. 1, 18 or 19 includes proteins having an amino acid sequence with one or more amino acids substituted, deleted or added and having the enzymatic activity of hydroxylating the 4-position of L-proline. The substitution, the deletion and the addition of amino acids can be conducted in accordance with the methods described in Nucleic Acids Research, 10, 6487 (1982); Proc. Natl. Acad. Sci. USA., 79, 6409 (1982); Proc. Natl. Acad. Sci. USA., 81, 5662 (1984); Science, 224, 1431 (1984); PCT WO85/00817 (1985); Nature, 316, 601(1985); Gene, 34, 315 (1985); Nucleic Acids Research, 13, 4431 (1985); Current Protocols in Molecular Biology, Chap. 8, Mutagenesis of Cloned DNA, John Wiley & Sons, Inc. (1989), etc.

The present invention encompasses any and every L-proline-4-hydroxylase gene of a DNA fragment containing a gene that codes for a protein having the enzymatic activity of hydroxylating the 4-position of L-proline, and this may include, for example, genes coding for the protein having the amino acid sequence as indicated by Sequence No. 1, 18 or 19, and also genes which code for a protein that has an amino acid sequence corresponding to the amino acid sequence as indicated by Sequence No. 1, 18 or 19 and derived therefrom by substitution, deletion or addition of at least one amino acid and which have the enzymatic activity of hydroxylating the 4-position of L-proline. Concretely mentioned are DNAs indicated by Sequence Nos. 2, 8 and 15, etc.

The L-proline-4-hydroxylase genes of the present invention include the DNAs as defined hereinabove and also DNAs as derived therefrom by mutation, such as substituting mutation, deleting mutation, inserting mutation or the like, to be conducted to the extent that the mutated DNAs do not lose the L-proline-4-hydroxylase activity, for example, DNAs with homology to Sequence No. 2, 8 or 15. Such homologous DNAs are those to be obtained by colony hybridization or plaque hybridization using, as a probe, the DNA having the nucleotide sequence as indicated by Sequence No. 2, 8 or 15. These treatments can be conducted in accordance with known in vitro recombination techniques [see Molecular Cloning: A Laboratory Manual, 2nd Ed., edited by Sambrook, Fritsch, Maniatis, published by Cold Spring Harbor Laboratory Press, 1989].

The DNA fragment containing the L-proline-4-hydroxylase gene can be obtained from microorganisms having the ability of hydroxylating L-proline to produce trans-4-hydroxy-L-proline. As the microorganism, any microorganism having the ability of hydroxylating L-proline to produce trans-4-hydroxy-L-proline can be employed in the present invention. As preferable examples of such a microorganism, microorganisms belonging to the genus Dactylosporangium, Amycolatpsis or Streptomyces and having the activity of L-proline-4-hydroxylase can be mentioned. More preferable examples thereof include Dactylosporangium sp. RH1 (FERM BP-4400), Amycolatpsis sp. RH2 (FERM BP-4581), Streptomyces griseovirides JCM4250, Streptomyces daghestanicus JCM4365, and mutants or derivatives of these strains.

Dactylosporangium sp. RH1 and Amycolatpsis sp. RH2 are microorganisms isolated by the present inventors isolated as those having the ability of producing L-proline-4-hydroxylase, and Streptomyces griseovirides JCM4250 and Streptomyces daghestanicus JCM4365 are microorganisms whose ability of producing L-proline-4-hydroxylases have been found by the present inventors for the first time.

Methods for obtaining L-proline-4-hydroxylase gene of the microorganism having the ability of producing L-proline-4-hydroxylase are described below.

Chromosomal DNA is prepared from a microorganism having the ability of producing L-proline-4-hydroxylase through a usual DNA isolation method, for example, a phenol method (Biochem. Biophys. Acta, 72, 619). The thus-obtained chromosomal DNA is cleaved with a suitable restriction endonuclease; then the restriction endonuclease cleaved fragments are inserted into vector DNAs to construct chromosomal DNA libraries for the chromosomes of the microorganisms. Using this chromosomal DNA library, a host microorganism can be transformed. The transformants containing the L-proline-4-hydroxylase gene are selected from the obtained transformants by a hybridization method. DNAs containing the intended gene can be obtained from the thus-selected transformants.

The process comprising a series of such steps can be conducted in accordance with known in vitro recombination method (molecular Cloning, A Laboratory Manual, 2nd edition, edited by Sambrook, Fritsch and Maniatis, Cold Spring Harbor Laboratory Press, 1989).

As the vector DNAs that are used to construct the chromosomal DNA library of the microorganism having the ability of producing L-proline-4-hydroxylase, phage vectors and plasmid vectors can be used if these can be replicated autonomously in Escherichia coli K12 strain. Preferable examples of the vector DNA include λ ZAPII, pUC18 and pBluescript (commercially available from STRATAGENE Co.).

As the host microorganisms that are used to construct the chromosomal DNA library of the microorganism having the ability of producing L-proline-4-hydroxylase, any of the microorganisms belonging to the genus Escherichia can be used. Preferable examples of the host microorganisms include E. coli XL1-Blue, E. coli XL2-Blue, E. coli DH1, E. coli MC1000, etc.

Based on the information about the amino acid sequence of L-proline-4-hydroxylase, DNA primers are synthesized. Using the DNA primers, DNA fragments are prepared through polymerase chain reaction (hereinafter referred to as PCR) . Using the thus-obtained DNA fragments, transformants containing an L-proline-4-hydroxylase gene can be selected by the hybridization method.

The information on the amino acid sequences of L-proline-4-hydroxylases can be obtained through analysis of pure L-proline-4-hydroxylases using ordinary amino acid sequencers, such as Protein Sequencer Model PPSQ-10 (produced by Shimadzu Seisakusho K.K.). As the information on the amino acid sequences thus obtained, concretely mentioned are partial amino acid sequences in the amino acid sequence as indicated by Sequence No. 1, for example, a partial amino acid sequence having the amino acid sequence from the N-terminal to the 24th amino acid sequence indicated by Sequence No. 1, etc.

The DNA primer can be synthesized by means of an ordinary DNA synthesizer, for example, 380A·DNA Synthesizer manufactured by Applied Biosystems.

As the probes for the hybridization, usable are partial fragments of L-proline-4-hydroxylase genes, which can be obtained through PCR. For example, a DNA as indicated by Sequence No. 3 (this corresponds to a sense chain DNA coding for the first to the sixth amino acids in the amino acid sequence of Sequence No. 1) and a DNA as indicated by Sequence No. 4 (this corresponds to an anti-sense chain DNA coding for the 19th to 24th amino acids in the amino acid sequence of Sequence No. 1) are chemically synthesized. Through PCR using these as DNA primers, obtained is a DNA fragment of 71 bp as indicated by Sequence No. 5. The thus-obtained DNA fragment can be used as the probe for the hybridization.

The DNA which contains the L-proline-4-hydroxylase gene and which is obtained from the transformant selected by the hybridization, is cleaved by a suitable restriction endonucleases, for example, Xho I, and then cloned into a plasmid such as pBluescript KS(+) (commercially available from STRATAGENE Co.). The nucleotide sequence of the above-mentioned gene can be determined by ordinary nucleotide-sequence determination methods, for example, the dideoxy chain termination method of Sanger et al. [Proc. Natl. Acad. Sci., U.S.A., 74, 5463, (1977)]. The determination of the nucleotide sequence can be conducted by an automatic DNA sequencer, for example, 373A·DNA Sequencer of Applied Biosystems. As the thus-determined nucleotide sequences of the L-proline-4-hydroxylase genes, for example, the nucleotide sequence indicated by Sequence No. 2 and 8 can be mentioned.

The DNA that codes for an L-proline-4-hydroxylase of the present invention can be introduce into vectors in a usual manner.

As the plasmids containing the DNA encoding the L-proline-4-hydroxylase of the present invention, for example, pRH71, etc. can be mentioned. Escherichia coli SOLR/pRH71 which is Escherichia coli containing pRH71 was deposited at the National Institute of Bioscience and Human-Technology of the Agency of Industrial Science and Technology (which is located at 1-3, Higashi 1-chome, Tsukuba-shi, Ibaraki-ken 305, Japan) as of March 2, 1995 under FERM BP-5025 in terms of the Budapest Treaty.

To express the thus-obtained L-proline-4-hydroxylase gene in the host, the DNA fragment containing the L-proline-4-hydroxylase gene is first cleaved by a restriction endonuclease or other deoxyribonuclease to form a DNA fragment of a suitable length containing the L-proline-4-hydroxylase gene. The thus-formed DNA fragment is inserted into an expression vector at the downstream position of the promoter therein, and thereafter the expression vector having the thus-inserted DNA therein is introduced into a host cell suitable for the expression vector.

Any host cell that can express the intended gene can be used. As examples of the host cell, microbial cells of a microorganism belonging to the genus Escherichia, Serratia, Corynebacterium, Brevibacterium, Pseudomonas, Bacillus, etc., as well as yeast strains, animal cell hosts, etc. can be mentioned.

An expression vector, which can be autonomously replicable in the above-mentioned host cell or capable of being inserted into a chromosome and which contains a promoter at the position where the L-proline-4-hydroxylase gene can be transcribed, can be used.

When the microorganisms such as Escherichia coli or the like are used as the host cell, it is advisable that the expression vector is replicated autonomously in the microorganisms and is composed of a promoter, a ribosome binding sequence such as a Shine-Dargarnosequence, an L-proline-4-hydroxylase gene and a transcription termination sequence. A regulatory gene may be contained therein.

As examples of the expression vector, mentioned are pBTrp2, pBTac1, pBTac2 (all commercially available from Behringer Manheim Co.) ; pKYP10 (see Japanese Published Unexamined Patent Application No. 110600/83); pKYP200 [see Agric. Biol. Chem., 48, 669 (1984)]; pLSA1 [see Agric. Biol. Chem., 53, 277 (1989)]; pGEL1 [see Proc. Natl. Acad. Sci. USA., 82, 4306 (1985)]; pBluescript (produced by STRATAGENE Co.); pTrs30 [prepared from Escherichia coli JM109/pTrS30 (FERM BP-5407); pTrs32 [prepared from Escherichia coli JM109/pTrs32 (FERM BP-5408)], etc.

As the promoter, usable is any one capable of being expressed in hosts such as Escherichia coli. For example, mentioned are promoters derived from Escherichia coli, phage, etc., such as trp promoter (Ptrp), lac promoter (Plac), P_{L} promoter and P_{R} promoter. Also usable are artificially designed and modified promoters, such as Ptrpx2 to be prepared by connecting two Ptrps in series, as well as tac promoter (ptac).

As the ribosome-binding sequence, any one capable of being expressed in hosts such as Escherichia coil can be used. However, it is desirable to use plasmids having a ribosome-binding sequence and an initiation codon as spaced at suitable intervals therebetween (for example, by 6 to 18 bases).

The L-proline-4-hydroxylase gene includes any and every gene that codes for an L-proline-4-hydroxylase. However, it is desirable that the bases constituting the DNA sequence of the gene are suitably substituted in order that the substituted DNA sequence can be constituted of codon most suitable for expression in the host microorganisms to be used. As examples of L-proline-4-hydroxylase genes where the constitutive bases have been substituted to modify them into codons most suitable for their expression, mentioned are the nucleotide sequence of Sequence No. 15, etc.

Transcription terminator sequences are not always necessary for the expression of the genes of the present invention. However, it is desirable that a transcription terminator sequence is arranged just after the structural gene.

Examples of the host cells usable in the present invention include Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Bacillus subtilis, Bacillus amyloliquefacines, Brevibacterium immariophilum ATCC14068, Brevibacterium saccharolyticum ATCC14066, Brevibacterium flavum ATCC14067, Brevibacterium lactofermentum ATCC13869, Corynebacterium glutamicum ATCC13032, Corynebacterium acetoacidophilum ATCC13870, Microbacterium ammoniaphilum ATCC15354, etc.

When the yeast strain is used as the host cell, for example, YEp13 (ATCC37115), YEp24 (ATCC37051), YCp50 (ATCC37419), etc. can be used as the expression vector.

As the promoter, any one that can be expressed in the host cell of the yeast strain can be used. As examples of the promoters, promoters of glycolytic genes such as hexose kinase, gal 1 promoter, gal 10 promoter, heat shock protein promoter, MFα1 promoter, and CUP 1 promoter can be used.

As examples of the host cells, Saccharomyces cerevisae, Schizosaccharomyces pombe, Kluvveromyces lactis, Trichosporon pullulans, and Schwanniomyces alluvius, etc. can be mentioned.

When the animal cells are used as the host cell, for example, pcDNA I/Amp, pcDNA I and pcDM8 (all commercially available from Funakosi Co.), etc. can be used as the expression vector.

As the promoter, any one that can be expressed in the host cell of animal cells can be used. For example, a promoter of an IE (immediate early) gene of human CMV, etc. can be used. An enhancer of the IE gene of human CMV may be used together along with the promoter.

As examples of the host cells, Namalwa, HBT5637 (Japanese Published Unexamined Patent Application No. 299/88), COS-cell, CHO-cell, etc. can be used.

To introduce DNA into animal cells, any and every method capable of introducing DNA into animal cells can be employed herein. For example, employable are electroporation methods [see Miyaji et al., Cytotechnology, 3, 133 (1990)], calcium phosphate methods (see Japanese Published Unexamined Patent Application No. 227075/90), lipofection methods [see Philip L. Felgner, et al., Proc. Natl. Acad. Sci., USA, 84, 7413 (1987)], etc. The resulting transformants can be collected and cultivated in accordance with the methods described in Japanese Published Unexamined Patent Application Nos. 227075/90 and 257891/90.

The thus obtained transformant is cultivated by an ordinary cultivation method.

The medium for cultivating these microbial transformants such as Escherichia coli, yeast strains or the like may be any of natural media and synthetic media, so long as it contains carbon sources, nitrogen sources, inorganic salts, etc., and transformants are efficiently cultivated.

Any carbon sources that can be assimilated by the microorganisms may be used. Examples of the carbon source include carbohydrates such as glucose, fructose, sucrose, molasses containing these components, starch and starch hydrolyzates; organic acids such as acetic acid and propionic acid; and alcohols such as ethanol and propanol.

As the nitrogen sources, ammonia, ammonium salts of inorganic and organic acids such as ammonium chloride, ammonium sulfate, ammonium acetate and ammonium phosphate, other nitrogen-containing compounds, peptone, meat extracts, yeast extracts, corn steep liquor, casein hydrolyzates, soybean cakes, soybean cake hydrolyzates, cultured fermented cells, their digested products, etc. may be used.

As inorganic salts, potassium dihydrogen phosphate, dipotassium hydrogen phosphate, magnesium phosphate, magnesium sulfate, sodium chloride, ferrous sulfate, manganese sulfate, copper sulfate, calcium carbonate, etc. may be used.

The cultivation is conducted under aerobic conditions, for example, with shaking culture or submerged-aerial stirring culture. The temperature for the cultivation is 15 to 40°C. The period for the cultivation is usually 16 to 96 hours. During the cultivation, the pH of the medium is kept at 3.0 to 9.0. The pH is adjusted using inorganic or organic acids, alkaline solutions, urea, calcium carbonate, ammonia or the like.

L-Proline is suitably added to the media in such a manner that its concentration may be from 5 to 1000 mM, preferably from 20 to 200 mM, whereby the intended L-proline-4-hydroxylases can be produced more efficiently.

Antibiotics such as ampicillin, tetracycline or the like may be added to the medium during the cultivation, if required.

For the cultivation of the microorganisms which are transformed with the expression vector using the inducible promoter, inducers may be added to the medium, if required. For example, in cultivation of microorganisms transformed with the expression vector using lac promoter, isopropyl-β-D-thiogalactopyranoside (IPTG) may be added to the medium. In cultivation of microorganisms transformed with the expression vector using trp promoter, indoleacrylic acid (IAA) may be added to the medium.

As the medium for cultivating the transformants which are obtained by using the animal cells as a host cell, RPMI1640 medium and Eagle's MEM medium which are generally used or these culture media containing a fetal bovine serum can be used.

The cultivation of the cells is conducted in the presence of 5% CO₂. The temperature for the cultivation is preferably 35 to 37°C, and the period for the cultivation is usually 3 to 7 days.

L-proline is suitably added to the media in such a manner that its concentration may be from 5 to 1000 mM, preferably from 20 to 200 mM, whereby the intended L-proline-4-hydroxylases can be produced more efficiently.

Antibiotics such as kanamycin, penicillin or the like may be added to the medium during the cultivation, if required.

A considerable amount of L-proline-4-hydroxylase is produced and accumulated in the thus-cultivated transformants in comparison to the microorganism strain used as the gene source, such as Dactylosporangium sp. RH1 or the like. 'Thus, the isolation and purification of the enzyme or the production of trans-4-hydroxy-L-proline from L-proline using the enzyme can be performed far more efficiently in comparison to the production of trans-4-hydroxy-L-proline from L-proline using the non genetically-engineered microorganism as the gene source, such as Dactylosporangium sp. RH1 or the like.

The formation of L-proline-4-hydroxylase in the transformants can be confirmed by detecting trans-4-hydroxy-1-proline produced by adding the culture, the cells or the treated cells to an aqueous medium suitable for the enzymatic reaction together with L-proline, a divalent iron ion and 2-ketoglutaric acid, and adding a surfactant or an organic solvent, if required, to determine trans-4-hydroxy-L-proline produced. With respect to the activity of the L-proline-4-hydroxylase of which the formation is confirmed in the cell, the activity of the enzyme for producing 1 nmol of trans-4-hydroxy-L-proline for 1 minute under the following conditions is defined as 1 unit (U). The microorganism cells and the animal cells are here called cells.

### Measurement of L-proline-4-hydroxvlase activity:

The cells, the treated cells or the enzyme preparation are added to 240 mM MES [2-(N-morphorino)ethanesulfonic acid] buffer (pH 6,5) containing 12 mM L-proline, 24 mM 2-ketoglutaric acid, 4 mM ferrous sulfate and 8 mM L-ascorbic acid to make 250 µl in total. The mixture is kept at 35°C for 10 minutes. The reaction mixture is heated at 100°C for 2. minutes to stop the reaction, and the amount of trans-4-hydroxy-L-proline produced in the reaction mixture is determined by high-performance liquid chromatography (hereinafter referred to as HPLC).

To determine the amount of trans-4-hydroxy-L-proline formed, any method capable of determining it can be employed. For example, employable are a method of separating and eluting trans-4-hydroxy-L-proline from the reaction mixture by means of HPLC using a ligand exchange chromatography column, such as SUMICHIRAL OA5000 (produced by Sumika Analysis Center Co.), or the like, followed by reacting it with 7-chloro-4-nitrobenz-2-oxa-1,3-diazole (hereinafter referred to as NBD) to give its derivative after the column and detecting the resulting derivative (post-column derivative method); a method of previously reacting the product in the reaction mixture with NBD to give its derivative therein followed by isolating and detecting the resulting derivative with NBD by means of reversed-phase chromatography with HPLC (pre-column derivative method) [see William J. Lindblad and Robert F. Diegelmann, Analytical Biochemistry, Vol. 138, pp. 390-395, 1984], etc. The detection of the derivative with NBD is conducted by measuring its luminescence (at the excited wavelength of 503 nm and the fluorescent wavelength of 541 nm). in both cases.

The enzyme may be isolated and purified in a usual manner from the culture of the transformant in which the formation of L-proline-4-hydroxylase is confirmed in the cultivated cell as mentioned above. For instance, the culture broth of the transformant is centrifuged to collect the cultivated cells therefrom, and the cells are washed and then disrupted by an ultrasonic cell disrupter, a French press, a Manton-Gauline homogenizer, a Dyno mill or the like to obtain a cell-free extract. The purified enzyme prepatarion can be obtained by ammonium sulfate precipitation, anion exchange chromatography such as diethylaminoethyl (DEAE) Sepharose or the like, hydrophobic chromatography such as butyl-Sepharose, phenyl-Sepharose or the like, gel filtration, electrophoresis such as isoelectric point electrophoresis, and so on from the supernatant of the cell-free extract obtained by centrifugation.

The cultivated transformant cells that have been identified to contain the L-proline-4-hydroxylase as formed therein can be cultivated under the same conditions as above, under which the transformant cells were cultivated, to thereby make the cells produce and accumulate trans-4-hydroxy-L-proline in the cells, and the thus-produced trans-4-hydroxy-L-proline can be collected from the culture to obtain it.

If the transformant cell derived from host cell which has the ability of producing L-proline from saccharide sources and accumulating it in the cultures and where such cells are used, it is possible to produce trans-4-hydroxy-L-proline even if L-proline is not added to the media during the cultivation of the cells therein. However, it is desirable to suitably add to the media L-proline at a concentration of from 5 to 1000 mM, preferably from 20 to 200 mM, whereby the intended trans-4-hydroxy-L-proline can be produced more efficiently.

If the transformant cells have the ability of producing 2-ketoglutaric acid from saccharide sources and accumulating it in the cultures and where such cells are used, it is possible to produce trans-4-hydroxy-L-proline even if 2-ketoglutaric acid is not added to the media during the cultivation of the cells therein. Where such transformant cells are used, saccharide sources such as glucose may be suitably added to the media to make the cells produce and accumulate 2-ketoglutaric acid in the cultures, whereby the intended trans-4-hydroxy-L-proline can be produced more efficiently. Where, on the other hand, transformant cells not having the ability of producing 2-ketoglutaric acid from saccharide sources are used, 2-ketoglutaric acid may be added to the media during the incubation of the cells, if desired.

If desired, 2-ketoglutaric acid and divalent iron ions may be added to the media during the cultivation of the transformant cells.

To produce trans-4-hydroxy-L-proline, also employable is another method to be mentioned below using, as the enzyme source, the cultures of the transformant cells where the formation of L-proline-4-hydroxylases has been identified, the cells isolated from the cultures, or the products as obtained by processing the cells.

The method to produce trans-4-hydroxy-L-proline is as follows. The cultures of the transformant cells, the cells isolated from the culture, or the products as obtained by processing the cells are added to aqueous media suitable for enzymatic reaction, along with L-proline, divalent iron ions and 2-ketoglutaric acid and optionally with surfactants and organic solvents, thereby converting L-proline into trans-4-hydroxy-L-proline, and thereafter the resulting trans-4-hydroxy-L-proline is collected from the reaction mixtures to obtain it.

As examples of the processed cells, dried cells, lyophilized cells, surfactant-treated cells, enzymatically-treated cells, ultrasonically-treated cells, mechanically-ground cells, mechanically-compressed cells, solvent-treated cells, fractionated cell proteins, immobilized cells, immobilized materials obtained by processing their cells, etc. can be used. The enzyme preparation obtained by extraction from the cells having L-proline-4-hydroxylase activity, purified products of these enzymes, and immobilized products thereof can also be used.

As examples of the aqueous medium, water, buffers such as phosphates, carbonates, acetates, borates, citrates and tris-buffers, alcohols such as methanol and ethanol, esters such as ethyl acetate, ketones such as acetone, and amides such as acetamide can be mentioned.

As examples of the surfactant, cationic surfactants such as polyoxyethylene-stearylamine (for example, Nymeen S215 produced by Nippon Oils & Fats Co.), cetyltrimethylammonium bromide, Cation FB, Cation F2-40E, etc.; anionic surfactants such as sodium oleylamidosulfate, Newrex TAB, and Rapizole 80.; ampholytic surfactants such as polyoxyethylene-sorbitan monostearate (for example, Nonion ST221) or the like.; and also other tertiary amines PB, hexadecyldimethylamine, etc. can be mentioned. Any and every surfactant that promotes the reaction may be employed. The concentration of the surfactant is usually from 0.1 to 50 mg/liter, preferably from 1 to 20 mg/liter.

As examples of the organic solvent, toluene, xylene, aliphatic alcohols, benzene and ethyl acetate can be mentioned. The concentration of the organic solvent is usually from 0.1 to 50 µl/ml, preferably from 1 to 20 µl/ml.

The reaction may be conducted during the cultivation of the transformant having the activity of L-proline-4-hydroxylase, or may also be conducted after the completion of the cultivation, in the aqueous medium using the cells, the treated cells, the purified enzyme or the crude enzyme prepared from the culture.

The amount of the enzyme added to the reaction mixture is determined depending on the amount of the substrate used. Usually, it may be from 1, 000 to 10,000,000 U/liter, preferably from 10,000 to 3,000,000 U/liter of the aqueous medium. In case of using the cells or the treated cells of the microorganism; the concentration of wet cells is usually from 1 to 300 g/liter.

The reaction is usually conducted at a temperature from 15 to 50°C at a pH from 6.0 to 9.0 for 1 to 96 hours.

The concentration of L-proline used in the reaction may be from 1 mM to 2 M. L-Proline can be supplied by adding L-proline itself to the reaction mixture, or adding the culture of the microorganism which can produce and accumulate L-proline from sugar source. Further, if a microorganism having the ability of producing L-proline from a sugar source is used as the host microorganism of the transformant, L-proline produced from a sugar source by the host microorganism can be used in the reaction. That is, L-proline produced by the transformant derived from the host microorganism having the ability of producing L-proline is converted into trans-4-hydroxy-L-proline in the culture broth using L-proline-4-hydroxylase produced by the transformant, whereby trans-4-hydroxy-L-proline can be produced in the culture without the addition of L-proline.

The divalent iron ion is required for the reaction. This divalent iron ion is ordinarily used in a concentration of from 1 to 100 mM. Any divalent iron ion can be used so long as it contains divalent iron and it does not inhibit the reaction. As examples of the divalent iron ion, sulfates such as ferrous sulfate; chlorides such as ferrous chloride; ferrous carbide; and organic acid salts such as citrates, lactates and fumarates can be mentioned. When the divalent iron ion is contained in the cells, the treated cells or the reaction mixture, the divalent iron need not be added.

2-Ketoglutaric acid itself may be added to the reaction mixture or may be supplied from a precursor which can be converted into 2-ketaglutaric acid by the metabolic activity of the cells or the treated cells used. As examples of such a precursor, saccharides such as glucose; amino acids such as glutamic acid; and organic acids such as succinic acid can be mentioned. These compounds may be used singly or in combination.

Trans-4-hydroxy-L-proline is recovered from the culture or the aqueous medium by any ordinary separation method, for example, column chromatography using an ion-exchange resin, etc. by crystallization, etc.

The structure of the thus-recovered trans-4-hydroxy-L-proline can be identified by ordinary analytical method such as ¹³C-NMR spectrum, ¹H-NMR spectrum, mass spectrum, specific rotation or the like.

The trans-4-hydroxy-L-proline produced by the present invention can be determined quantitatively by the above-mentioned post-column derivatization method or pre-column derivatization method.

### Brief Description of Drawings

Fig. 1 shows a restriction enzyme map of plasmid pRH71 and the steps of constructing plasmids pYan10 and pYan13.

In the figure, the thick, shadowed lines each indicate a cloned Dactylosporangium sp. RH1 chromosome site. Ap indicates a pBR322-derived ampicillin-resistant gene. In the figure, only the restriction enzyme sites having relation to the construction of the plasmids are shown.

Fig. 2 shows the steps of constructing plasmid pTr14.

In the figure, the thick, solid black lines each indicate a part that contains an L-proline 4-hydroxylase gene. Ap indicates a pBR322-derived ampicillin-resistant gene; and Ptrp indicates a promoter of Escherichia coli tryptophan operon. The arrows each indicate the direction in which the gene is transcribed and translated. In the figure, only the restriction enzyme sites having relation to the construction of the plasmid are shown.

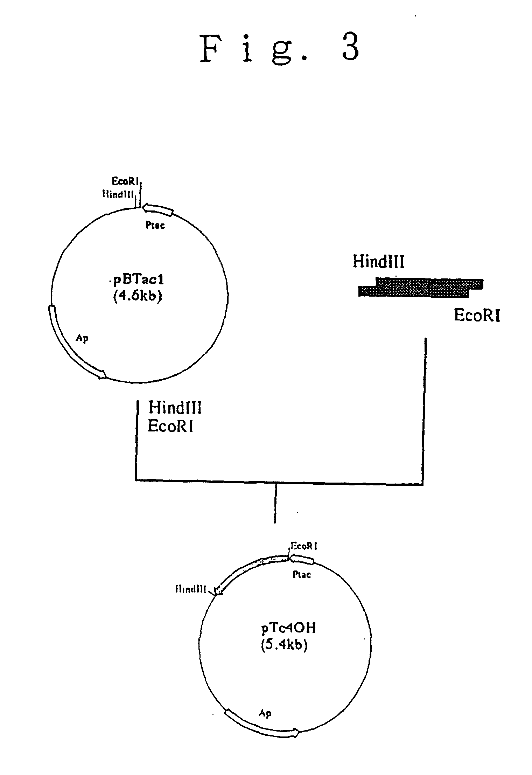

Fig. 3 shows the steps of constructing plasmid pTc4OH.

In the figure, the thick, solid black lines each indicate a part that contains an L-proline 4-hydroxylase gene. Ap indicates a pBR322-derived ampicillin-resistant gene; and Ptac indicates tac promoter. The arrows each indicate the direction in which the gene is transcribed and translated. In the figure, only the restriction enzyme sites having relation to the construction of the plasmid are shown.

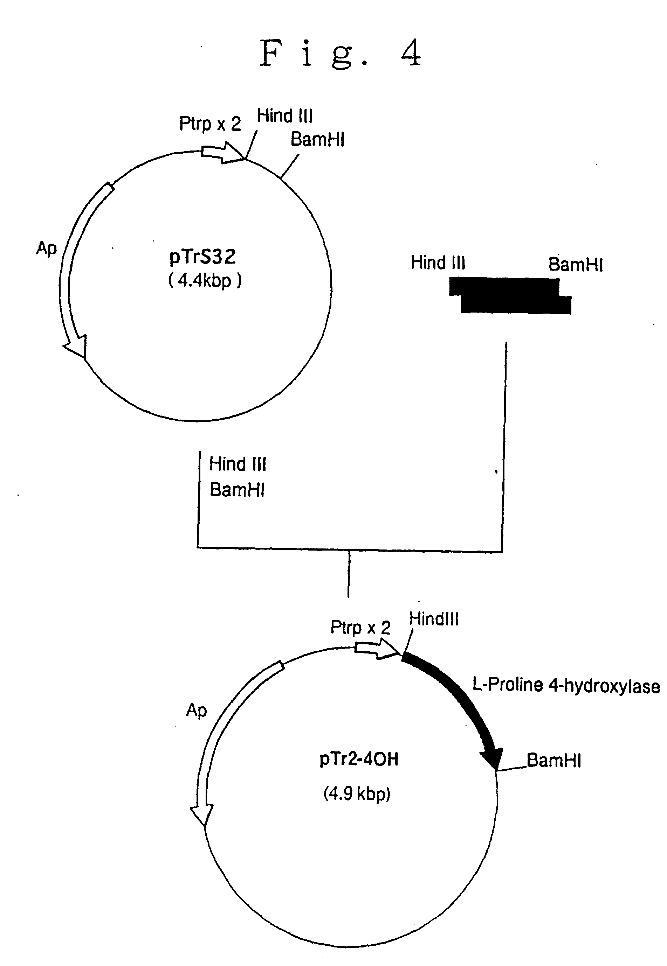

Fig. 4 shows the steps of constructing plasmid pTr2-40H.

In the figure, the thick, solid black line each indicate a part that contains an L-proline 4-hydroxylase gene. Ap indicates a pBR322-derived ampicillin-resistant gene; and Ptrpx2 indicates a promoter composed of two promoters of Escherichia coli-derived tryptophan operon as connected in series (tandem tryptophan promoter). The arrows each indicate the direction in which the gene is transcribed and translated. In the figure, only the restriction enzyme sites having relation to the construction of the plasmid are shown.

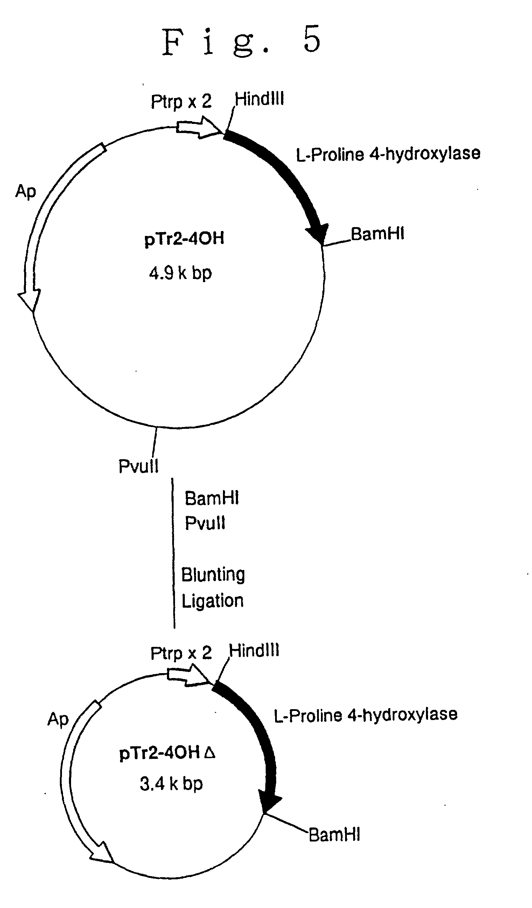

Fig. 5 shows the steps of constructing plasmid pTr2-4OHΔ.

In the figure, the thick, solid black lines each indicate a part that contains an L-proline 4-hydroxylase gene. Ap indicates a pBR322-derived ampicillin-resistant gene; and Ptrpx2 indicates a promoter composed of two promoters of Escherichia coli-derived tryptophan operon as connected in series (tandem tryptophan promoter). The arrows each indicate the direction in which the gene is transcribed and translated. In the figure, only the restriction enzyme sites having relation to the construction of the plasmid are shown.

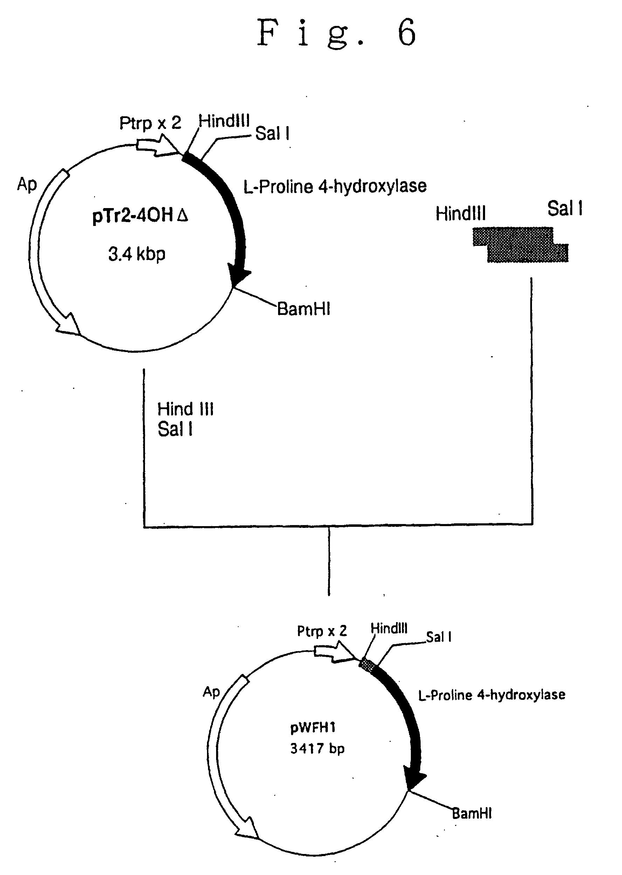

Fig. 6 shows the steps of constructing plasmid pWFH1.

In the figure, the thick, shadowed lines each indicate a site into which a PCR-amplified fragment as treated with HindIII and SalI is inserted. The thick, solid black lines each indicate a part that contains a Dactylosporangium sp. RH1-derived L-proline 4-hydroxylase gene. Ap indicates a pBR322-derived ampicillin-resistant gene; and Ptrpx2 indicates a promoter composed of two promoters of Escherichia coli-derived tryptophan operon as connected in series (tandem tryptophan promoter). The arrows each indicate the direction in which the gene is transcribed and translated. In the figure, only the restriction enzyme sites having relation to the construction of the plasmid are shown.

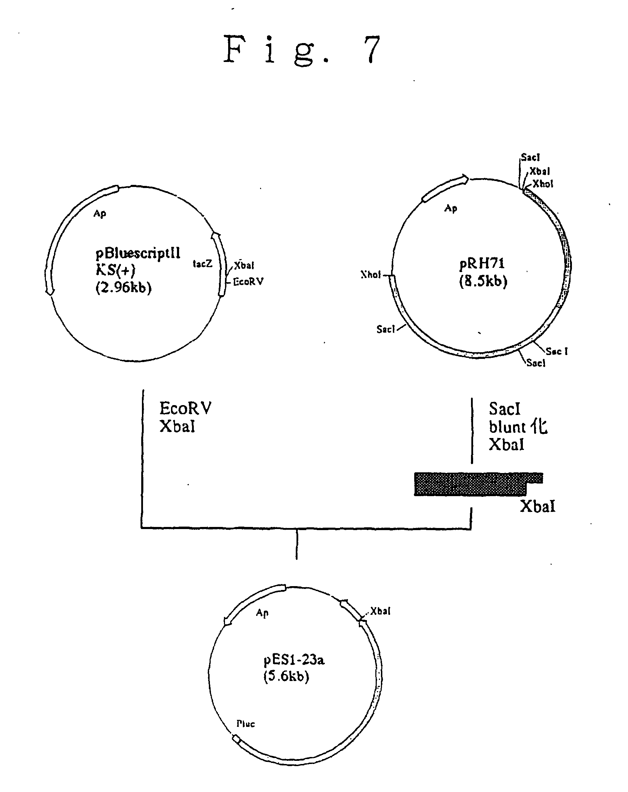

Fig. 7 shows the steps of constructing plasmid pES1-23a.

In the figure, the thick, solid black lines each indicate a part that contains an L-proline 4-hydroxylase gene. lacZ indicates Escherichia coli β-galactosidase gene; Ap indicates a pBR322-derived ampicillin-resistant gene; and Plac indicates lac promoter. The arrows each indicate the direction in which the gene is transcribed and translated. In the figure, only the restriction enzyme sites having relation to the construction of the plasmid are shown.

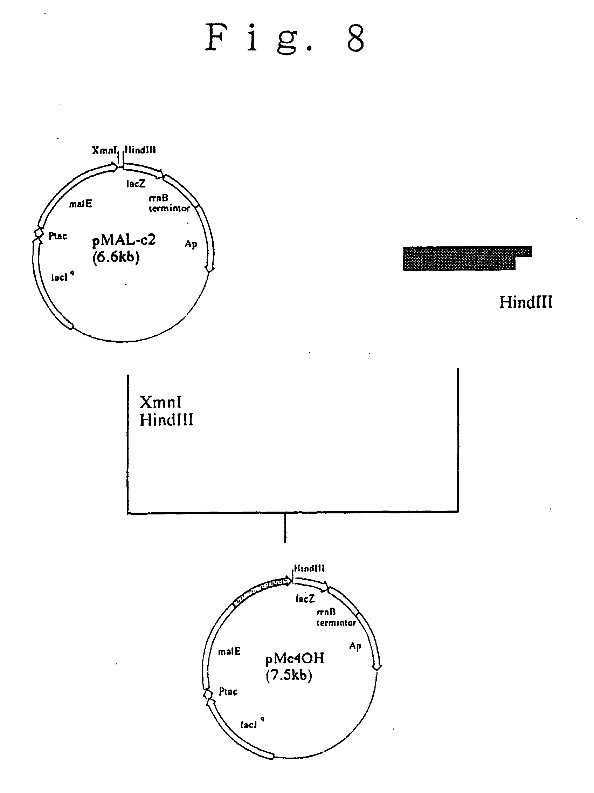

Fig. 8 shows the steps of constructing plasmid pMc4OH.

In the figure, the thick, solid black lines each indicate a part that contains an L-proline 4-hydroxylase gene. malE indicates Escherichia coli maltose-binding protein gene; lacZ indicates Escherichia coli β-galactosidase gene; Ap indicates a pBR322-derived ampicillin-resistant gene; lacIq indicates a repressor gene of Escherichia coli lactose operon; rrnB terminator indicates a terminator of rrnB gene; and Ptac indicates tac promoter. The arrows each indicate the direction in which the gene is transcribed and translated. In the figure, only the restriction enzyme sites having relation to the construction of the plasmid are shown.

### BEST MODES OF CARRYING OUT THE INVENTION

### Example 1 : Preparation of partial DNA fragment of the gene encoding L-proline-4-hydroxylase protein derived from Dactylosporangium sp. RH1 :

### (1) Isolation of Chromosomal DNA of Dactylosporangium sp. RH1:

Chromosomal DNA of Dactylosporangium sp. RH1 was isolated in the usual manner as follows. SK#2 medium (comprising 0.25 % glucose, 1.0 % soluble starch, 0.25% yeast extract, 0.25 % peptone, 0.15 % meat extract, 0.01 % potassium dihydrogen phosphate and 0.03 % magnesium sulfate, and adjusted to pH 7.6 with 6N NaOH) containing 5 % mannitol and 0.05 % glycine, was put into test tubes in an amount of 10 ml each, and sterilized at 120°C for 20 minutes. One loopfui of cells of Dactylosporangium sp. RH1 which had grown in HT-agar plate medium (comprising 1 % soluble starch, 0.2 % NZ amine, 0.1 % yeast extract, 0.1 % meat extract and 1.5 % agar, adjusted to pH7.2 with 6N NaOH and sterilized at 120°C for 20 minutes), was inoculated in the above-mentioned medium, and cultivated at 28°C for 3 days with shaking.

The culture was centrifuged, and the obtained cells were washed with 10 ml of a 10.3 % sucrose solution, and suspended in 6 ml of TS comprising 10.3 % sucrose, 50 mM tris·HCl (pH 8.0) and 25 mM EDTA. One milliliter of a lysozyme solution (50 mg/ml·TS) was added thereto, and the mixture was incubated at 37°C for 60 minutes. Subsequently, 0.6 ml of a Proteinase K (produced by Sigma Co.) solution (2 mg/ml·TS) was added to the lysozyme-treated solution, and gently stirred. Further, 3.6 ml of a 3.3% (w/v) SDS solution was added thereto while gently mixing, and the mixture was incubated at 37°C for 60 minutes. The mixture was heated at 50°C for 30 minutes, and then cooled with water. An equal amount of TE [containing 10 mM tris·HCl (pH 8.0) and 1 mM EDTA] saturated phenol-chloroform (1/1, v/v) was added thereto, and the mixed solution was moderately shaked for 30 minutes. After the centrifugation, the upper layer was taken, and again subjected to extraction with the mixture of TE saturated phenol-chloroform. The extract was centrifuged, and an equal amount of chloroform was then added to the upper layer, and mixed. The mixture was recentrifuged. The upper layer was taken, and 20 µl of an RNase A aqueous solution (10 mg/ml) heat-treated at 100°C for 10 minutes was added to the upper layer. The mixture was incubated at 37°C for 45 minutes. To the RNase A-treated solution were added 1/10 volume of a 5 M NaCl aqueous solution and 1/4 volume of 50 % PEG6000, and gently mixed. The mixture was allowed to stand overnight while being cooled with ice. After the mixed solution was centrifuged at 12, 000 rpm for 10 minutes, the supernatant was discarded completely, and the precipitate was dissolved in 5 ml of TE. After 1/10 volume of a 3 M sodium acetate solution and 1/30 volume of a 66 mM magnesium chloride solution were added thereto and mixed, 2.2-fold volumes of cold ethanol was added, and gently mixed. After the mixed solution was centrifuged at 10,000 rpm for 10 minutes, the supernatant was discarded, and the precipitate was washed twice with 70 % cold ethanol. The precipitate containing 250 µg of chromosomal DNA was dissolved in TE and used in the subsequent experiment as chromosomal DNA.

### (2) Determination of Partial Amino Acid Sequence of Dactylosporangium sp. RH1-derived L-Proline 4-Hydroxylase Protein:

L-proline 4-hydroxylase as produced by Dactylosporangium sp. RH1 was isolated and purified in accordance with the process of Reference Example 1, and the N-terminal amino acid sequence of the purified enzyme protein was sequenced, using Protein Sequencer Model PPSQ-10 (produced by Shimadzu Seisakusho Co.) to determine the N-terminal amino acid sequence thereof comprised of N-terminal 24 amino acids of the sequence of Sequence Number 1.

### (3) Preparation of partial DNA fragment of L-proline-4-hydroxylase gene:

The sense strand mixed DNA primer indicated in Sequence No. 3 corresponding to amino acids Nos. 1 to 6 of an amino acid sequence indicated in Sequence No. 1 and an anti-sense strand mixed DNA primer indicated in Sequence No. 4 corresponding to amino acids Nos. 19 to 24 indicated in Sequence No. 1 were synthesized using 380A· DNA Synthesizer manufactured by Applied Biosystems.

Using the above-synthesized DNA primers and Dactylosporangium sp. RH1 chromosomal DNA as a template, the PCR was conducted by a Program Temp Control System PC-700 manufactured by K.K. Astec. The reaction was conducted using 20 µl of a reaction mixture having the following formulation.

Formulation of the reaction mixture: 22 ng/µl Dactylosporangium sp. RH1 chromosome DNA, 10 µM sense chain mix DNA primer, 10 µM anti-sense chain mix DNA primer, 0.125 U/µl Pfu DNA polymerase (produced by STRATAGENE Co.), 10 % DMSO, 20 mM Tris-HCl (pH 8.2), 10 mM KCl, 6 mM ammonium sulfate, 2 µM magnesium chloride, 0.1% Triton X-100, 10 ng/µl bovine serum albumin.

After the completion of an incubation at 96°C for 5 minutes, a three step incubation, namely at 96°C for 2 minutes, at 37°C for 1 minute and at 72°C for 1 minute was repeated for a total of five times. Further, a three step incubation, namely at 96°C for 2 minutes, at 50°C for 1 minute and at 72°C for 1 minute was repeated for a total of 35 times. The reaction mixture was subjected to electrophoresis with 15% polyacrylamide (PAGEL NPU-15L produced by Atto Co.), and a band of 71 bp was recovered using da Vinci Kun (Pen Touch Recovery NB-7000 Model) manufactured by Nippon Eido K.K. The recovered DNA fragment (71 bp) was inserted into a Sma I site of pUC18 using a Sure Clone Ligation Kit produced by Pharmacia Co., and the nucleotide sequence was determined by a nucleotide sequencing kit (Taq DyeDeoxy™ Terminator Cycle Sequencing Kit produced by Applied Biosystems) . The determined nucleotide sequence of the DNA fragment of 71 bp is shown in Sequence No. 5. The amino acid sequence presumed from the nucleotide sequence of the DNA fragment of 71 bp completely agreed with the N-terminal amino acid sequence of the purified enzyme indicated in Sequence No. 1.

### Example 2': Cloning of a DNA fragment containing L-proline-4-hydroxylase gene:

### (1) Preparation of a DIG probe:

A digoxigenin (DIG) labeled DNA fragment of 71 bp was prepared using a PCR DIG Labelling Kit produced by Boehringer Mannheim.

The PCR was conducted using 2.5 U of Pfu DNA Polymerase (produced by Stratagene), 5 µl of x10 Buffer for Pfu DNA polymerase (produced by Stratagene) , 5 µl of DM50, 5 µl of x10 PCR DIG Mix (produced by Boehringer Mannheim), 1 µl of a dilute solution obtained by diluting to ten times the DNA solution containing the fragment of 71 bp formed in the PCR and recovered after the electrophoresis with polyacrylamide gel in (3) of Example 1, and 50 µl of a reaction mixture containing 10 µM of the sense strand synthetic DNA indicated in Sequence No. 3 and 10 µM of the anti-sense strand synthetic DNA indicated in Sequence No. 4. After the completion of an incubation at 96°C for 5 minutes, a three step incubation, namely at 96°C for 2 minutes, at 50°C for 1 minute and at 72°C for 1 minute was repeated to a total of 35 times. The reaction mixture was subjected to electrophoresis with 12.5 % polyacrylamide gel to identify the formation of an amplification fragment of 71 bp. The fragment was recovered from the gel in the same manner as in (3) of Example 1, and used as a probe.

### (2) Southern hybridization:

Restriction endonuclease Xho I (produced by Takara Shuzo, 36 U) was added to 10 µg of chromosomal DNA of Dactylosporangium sp. RH1, and the mixture was incubated at 37°C for 2 hours. DNA was cleaved, and subjected to electrophoresis with agarose gel using the probe obtained in (1) of Example 2 and DIG Luminescent Detection Kit produced by Boehringer Mannheim, and Southern hybridization was conducted according to the method described in a manual attached to the Kit.

That is, after the completion of the agarose gel electrophoresis, the agarose gel was shaked gently in 0.25 N hydrochloric acid for 20 minutes, and then dipped in a mixture of 0.5 M sodium hydroxide and 1.5 M sodium chloride for 50 minutes and further in a mixture of 2 M sodium chloride and 1M tris·HCl (pH 5.0) for 25 minutes. While sucking the gel at 7.5 mmHg by means of a Genopirator Pump AE-6680P produced by Atto Co. and a Genopirator AE-6680C also produced by Atto Co., Hybond-N⁺ Film (produced by Amersham) was blotted with the gel in SSC at a concentration of 20 times (formulation of SSC at a concentration of 1 time - 150 mM sodium chloride and 15 mM sodium citrate). After the completion of the blotting, the film was dried at 80°C for 10 minutes, and then crosslinked using FUNA-UV-LINKER FS-800 (produced by Funakoshi) . The thus-obtained film was dipped in 10 ml of a hybridization buffer (solution obtained by dissolving 50 % v/v formamide, 2 % blocking reagent, 0.1 % w/v N-laurylsarcosine and 0.02 % w/v SDS in SSC at a concentration of 5 times) of a DIG Luminescent Detection Kit at 42°C for 1 hour, and then dipped in a probe solution [obtained by adding 3 µl of the probe obtained in (1) of Example 2 to 200 µl of a hybridization buffer, treating the mixture at 95°C for 2 minutes, and then adding the hybridization buffer to adjust the total amount to 1.5 ml] overnight at 42°C. The thus-obtained film was further washed twice with 25 ml of SSC containing 0.1 % SDS at a concentration of 2 times at room temperature for 5 minutes each, and then washed twice with 25 ml of SSC containing 0.1 % SDS at a concentration of 0.1 time at 68°C for 15 minutes each.

The thus-washed film was treated with a washing buffer [Buffer 1 ( 0.1 M maleic acid, 0.15 M sodium chloride, pH 7.5) containing 0.3 % w/v Tween-20] at room temperature for 1 to 5 minutes, with 50 ml of Buffer 2 (Buffer 1 containing 1 % blocking reagent) at room temperature for 30 minutes, with 10 ml of Buffer 2 containing 1 µl anti-digoxigenin-AP Fab at room temperature for 30 minutes, twice with 50 ml of Buffer 2 at room temperature for 30 minutes each, with 10 ml of Buffer 3 (a buffer containing 0.1 M tris·HCl, 0.1 M sodium chloride and 50 mM magnesium chloride, pH 9.5) at room temperature for from 2 to 5 minutes, and with 5 ml of Buffer 3 containing 50 µl of Lumigen PPD at room temperature for 5 minutes in this order. Subsequently, water was removed from the film quickly over a filter paper, wrapped with Saran Wrap, and then allowed to stand at 37°C for 15 minutes. The resulting film was exposed at room temperature for 30 minutes using a Hyperfilm-ECL (produced by Amersham).

It was found that the DNA fragment which had hybridized strongly with the probe was present at a position of approximately 5.5 kb.

### (3) Fractionation of chromosomal DNA:

Restriction endonuclease Xho I (produced by Takara Shuzo Co., Ltd., 360 U) was added to 100 µg of chromosomal DNA of Dactylosporangium sp. RH1, and the mixture was incubated at 37°C for 2 hours. After DNA was cleaved, an equal amount of a mixture of TE saturated phenol-chloroform was added thereto, and mixed. After the mixture was centrifuged, the upper layer was taken, and mixed gently with 2.2-fold volumes of cold ethanol. The mixture was centrifuged at 10,000 rpm for 10 minutes. After the supernatant was discarded, the precipitate was washed twice with 70 % cold ethanol to obtain an ethanol precipitate (hereinafter the procedure for obtaining the ethanol precipitate using the mixture of TE saturated phenol-chloroform mixture and the cold ethanol referred to as "ethanol precipitation"). The precipitate was dissolved in 120 µl of TE, and the mixture was subjected to agarose gel electrophoresis. After the completion of the electrophoresis, a DNA fraction of approximately 5.5 kb was extracted from the agarose gel and purified using Prep-A-gene (produced by Biorad Co.) to obtain approximately 7 µg of the Xho I cleaved chromosomal DNA fraction.

### (4) Construction of phage library:

Using an undigested λ ZAPII Cloning Kit produced by Stratagene, a phage library was formed as follows.

Restriction endonuclease Xho I (produced by Takara Shuzo Co., Ltd., 36 U) was added to 5 µg of λ ZAPII DNA, and the mixture was incubated at 37°C for 3 hours. After DNA was cleaved, the ethanol precipitate was obtained by ethanol precipitation. After the ethanol precipitate was dissolved in 35 µl of TE, the solution was dephosphorylated using Alkaline Phosphatase (Calf Intestine) produced by Takara Shuzo Co., Ltd. according to the method described in a manual attached thereto. After the completion of the dephosphorylation, the ethanol precipitate was obtained by the ethanol precipitation, and the precipitate was dissolved in 5 µl of TE. The thus-obtained Xho I-cleaved λ ZAPII DNA (0.36 µg) was reacted with 0.35 µg of Xho I-cleaved chromosomal DNA obtained in (3) of Example 2 at 26°C for 2.5 hours using a ligation kit (TAKARA Ligation Kit produced by Takara Shuzo Co., Ltd.) to ligate the two DNAs. Ethanol was added to the reaction mixture, and the resulting DNA precipitate was dissolved in 4 µl of TE. The DNA was further packaged in λ phage particles using a Gigapack II Gold Packaging Extract produced by Stratagene.

Meanwhile, E. coli XL1-Blue MRF' strain (produced by Stratagene) was inoculated in 3 ml of LB medium (solution obtained by dissolving 10 g of bactotryptone, 5 g of bactoyeast extract and 5 g of NaCl in 1 liter of distilled water and sterilized at 120°C for 20 minutes) containing 0.2 % (w/v) maltose and 10 mM magnesium sulfate, and cultivated at 30°C for 16 hours. After the completion of the cultivation, the cells were collected by centrifugation, and suspended in 10 mM of a sterilized magnesium sulfate solution such that the absorbance at 600 nm was approximately 0.5.

Two-hundred microliters of the above-obtained cell solution was mixed with 10 µl of a packaging solution, and the mixed solution was then incubated at 37°C for 15 minutes. To the mixed solution were added 3 ml of LB soft agar medium (obtained by adding an agar to LB medium such that the amount of the agar was 0.6 %) 15 µl of a 0.5 M IPTG aqueous solution and 50 µl of X-Gal (5-bromo-4-chloro-3-indolyl-B-D-galactoside) solution (250 mg X-Gal/ml·dimethylformamide). The mixture was put on LB agar medium (obtained by adding an agar to LB medium such that the amount of the agar was 1.8 %), and cultivated overnight at 37°C.

Approximately 5, 000 colorless plaques were obtained and used as a phage library.

### (5) Selection of an intended clone:

The plaque having the intended clone was selected from the above-mentioned phage library as follows.

The plaques appearing on the LB agar medium were shifted onto a nylon film (Nytran produced by Schleicher & Schuell) washed with SSC having a 5-fold concentration. Subsequently, the film was allowed to stand on a filter paper immersed with 0.5 M sodium hydroxide and 1.5 M sodium chloride for 5 minutes. Further, the film was allowed to stand on a filter paper immersed with 1.5 M sodium chloride and 0.5 M tris·HCl (pH 8.0) twice for 2 minutes each and on a filter paper immersed with SSC having a 2-fold concentration for 2 minutes each. Subsequently, the resulting film was dried at 80°C for 30 minutes. The dried film was washed with SSC containing 0.1 % SDS having a 2-fold concentration and then with SSC having a 2-fold concentration, and air-dried.

The detection was conducted using the DIG probe obtained in (1) of Example 2 and a DIG Luminescent Detection Kit produced by Boehringer Mannheim according to the method described in (1) of Example 2. Consequently, one positive plaque having the intended clone was detected.

### (6) Identification of a clone:

A portion of approximately 1 cm² around the positive plaque was cut out. One milliliter of SM (a medium containing 5.8 g/l sodium chloride, 2 g/l magnesium chloride, 0.01 % gelatin, and 50 mM tris·HCl, pH 7.5) and 20 µl of chloroform were added thereto. The mixture was stirred thoroughly, and then centrifuged. The thus-obtained supernatant was used as a phage extract.

A sense strand DNA primer indicated in Sequence No. 6 corresponding to Nucleotide Nos. 13 to 32 of the nucleotide sequence described in Sequence No. 5 and an anti-sense strand DNA primer indicated in Sequence No. 7 corresponding to Nucleotide Nos. 53 to 71 of the nucleotide sequence indicated in Sequence No. 5 (provided that the'nucleotide corresponding to Nucleotide No. 66 indicated in Sequence No. 5 was G) were synthesized by means of 330A·DNA Synthesizer manufactured by Applied Biosystems.

Using 5 µl of the phage extract, the sense strand DNA primer indicated in Sequence No. 6 and the anti-sense strand DNA primer indicated in Sequence No. 7, the PCR was conducted according to the method described in (3) of Example 1 to obtain a DNA fragment of 59 bp. The DNA fragment was analyzed by electrophoresis with 12.5 % polyacrylamide, and identified as the intended clone.

The procedures described in (4) to (6) of Example 2 were repeated except that the above-obtained phage extract was used instead of the packaging liquid shown in (4) of Example 2 to purify the intended clone.

### (7) Formation of a plasmid by in vivo excision of the phage DNA:

Formation of a plasmid by in vivo excision of the phage DNA in the extract obtained in (6) of Example 2 was conducted using an undigested λ ZAPII Cloning Kit produced by Stratagene according to a method described in a manual attached thereto as described below.

E. coli XL1-Blue MRF' strain was inoculated in 3 ml of LB medium containing 0.2 % (w/v) maltose and 10 mM magnesium sulfate according to a method described in (4) of Example 2 and was cultivated at 30°C for 16 hours. After the completion of the cultivation, the culture was centrifuged, and the obtained cells were suspended in 10 mM magnesium sulfate solution such that the absorbance at 600 nm was approximately 1.0. To 200 µl of the cell suspension were added 100 µl of the phage extract obtained in (6) of Example 2 arid 1 µl of an ExAssist Helper Phage (produced by Stratagene), and the mixture was incubated at 37°C for 15 hours. To this reaction mixture was added 3 ml of 2xYT (obtained by dissolving 10 g of sodium chloride, 10 g of yeast extract and 16 g of bactotryptone in 1 liter of distilled water, and sterilized at 120°C for 20 minutes), and the mixture was shaken at 37°C for 2 hours. The resulting solution was heated at 70°C for 20 minutes, and centrifuged to obtain a supernatant. One microliter of the supernatant was added to 200 µl of suspension of E. coli SOLR strain cultivated in the same manner as E. coli XL1-Blue MRF' strain. The mixture was incubated at 37°C for 15 minutes, then spread on LB agar medium containing 50 µg/ml of ampicillin, and cultivated overnight at 37°C. A positive colony was selected from the colonies grown on the agar medium according to the method described in (6) of Example 2 except that the colonies were used instead of the phage extract.

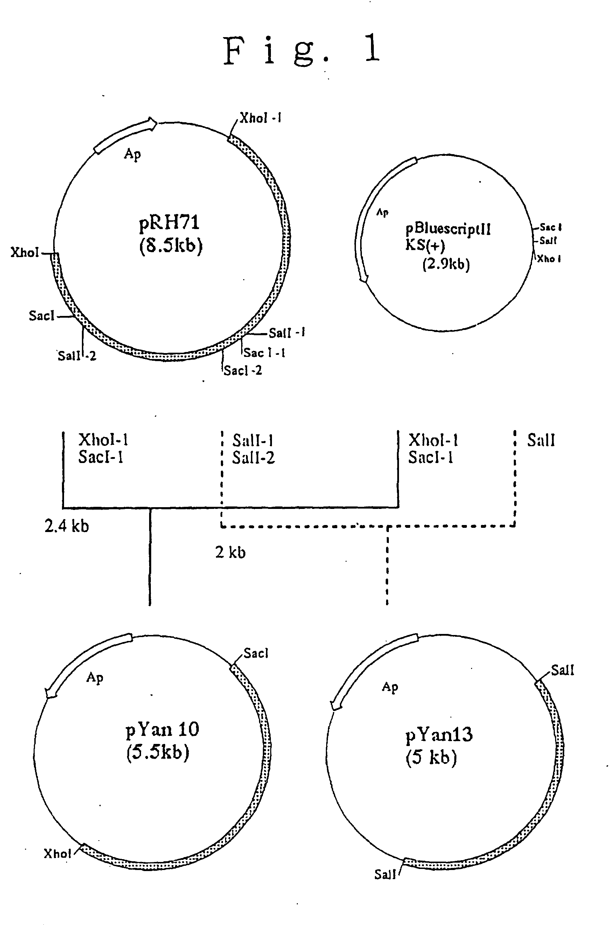

A plasmid was extracted from the thus-obtained positive colony in a usual manner, and the structure thereof was identified by digestion with restriction endonucleases. The thus-obtained plasmid pRH71 had a structure in which the Xho I-cleaved DNA fragment having a size of approximately 5.5 kb was inserted in the Xho I site of pBluescript SK(-) as shown in Fig. 1.

### (8) Determination of nucleotide sequence:

From the Xho I fragment having a size of approximately 5.5 kb inserted in the above-obtained pRH71, a Sac I-Xho I fragment having a size of approximately 2.4 kb (fragment to be cleaved with Xho I-1 and Sac I-1 in Fig. 1) or a Sac I fragment having a size of approximately 2 kb (fragment to be cleaved with Sac I-1 and Sac I-2) was cleaved with the respective restriction endonucleases, and subcloned at the Sac I-Xho I cleavage site or the Sac I cleavage site of pBluescript KS (+) respectively to obtain respective plasmids pYan10 or pYan13 as shown in Fig. 1.

Using a deletion kit for kilosequences produced by Takara Shuzo Co., Ltd., deletion mutant plasmids were constructed from pYan10 according to a method described in a manual attached thereto.

A nucleotide sequence of the Sac I-Xho I fragment having the size of approximately 2.4 kb in the deletion plasmid was determined using a sequence determination kit (Taq DyeDeoXy™ Terminator Cycle Sequencing Kit produced by Applied Biosystems).

With respect to pYan13, deletion mutant plasmids were constructed, and the nucleotide sequence determination was conducted in the same manner as mentioned above to determine the nucleotide sequence of the Sac I-Sac I fragment (fragment to be cleaved with Sac I-1 and Sac I*-*2 in Fig. 1) in the Sac I fragment having the size of approximately 2 kb.

The nucleotide sequence of the Sac I-Xho I fragment (fragment to be cleaved with Xho I-1 and Sac I-2 in Fig. 1) 2707b is indicated in Sequence No. 8.

In the thus-determined nucleotide sequence, the nucleotide sequence (corresponding to Nucleotide Nos. 264 to 1079 in Sequence No. 8) represented by Sequence No. 2, which encodes a protein composed of 272 amino acids indicated in Sequence No. 1 was present. This amino acid sequence included the N-terminal amino acid sequence indicated in Sequence No. 1 which was determined using purified L-proline-4-hydroxylase. Thus, it was identified that the intended L-proline-4-hydroxylase gene was present in the obtained Xho I fragment having a size of approximately 5.5 kb.

### Example 3: Construction of an L-proline-4-hydroxylase expression plasmid:

### (1) Construction of an expression plasmid using trp promoter (Ptrp):

A sense strand DNA primer indicated in Sequence No. 9 and an anti-sense strand DNA primer indicated in Sequence No. 10 were synthesized by 380A·DNA Synthesizer manufactured by Applied Biosystems. The PCR was conducted using the synthetic DNA's as the primers and pRH71 as a template. The reaction was conducted in the same manner as in Example 1 using 20 µl of a reaction mixture containing 0.1 µg of pRH71, 2 µM sense strand DNA primer and 2 µM anti-sense strand DNA primer. That is, after incubation at 96°C for 5 minutes, a three step incubation, namely at 96°C for 2 minutes, at 58°C for 1 minute and at 75°C for 1 minute was repeated for a total of 30 times. The reaction mixture was subjected to agarose gel electrophoresis. After it was identified that an amplified fragment of 844 bp encoding the structural gene of L-proline-4-hydroxylase was formed, the amplified fragment was extracted from the agarose gel in a usual manner, and recovered using Prep-A-gene produced by Biorad Co. Both terminals of the DNA fragment of 844 bp recovered were cleaved with Hind III and Bam HI, and an ethanol precipitate was then obtained by the ethanol precipitation. The ethanol precipitate was dissolved in 5 µl of TE.

Plasmid pTrS30DNA containing Ptrp was cleaved by Hind III and Bam HI. The L-proline-4-hydroxylase structural gene fragment treated with Hind III and Barn HI was inserted into the cleavage site via the ligation kit produced by Takara Shuzo Co., Ltd. Using the thus-obtained plasmid, E. coli XL1-Blue MRF' strain was transformed in a usual manner. The transformant was spread on LB agar medium containing 50 µg/ml of ampicillin, and then cultivated overnight at 37°C. The plasmid was extracted from the colony of the grown transformant in a usual manner, and the structure of the plasmid was identified by digestion with a restriction endonuclease.

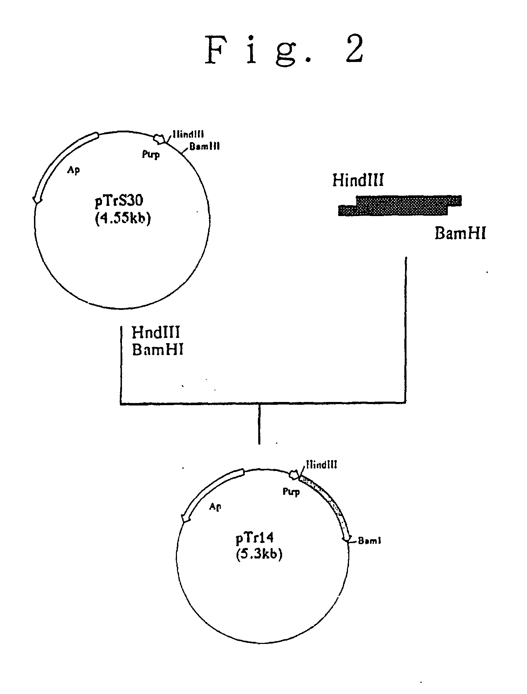

As a result, plasmid pTrl4 in which the DNA fragment containing the structural gene of L-proline-4-hydroxylase was inserted in the same direction as the transcription direction of Ptrp was obtained as shown in Fig. 2.

### (2) Construction of an expression plasmid using tac promotor (Ptac) :

An expression plasmid using Ptac was constructed in the same manner as in (1) of Example 3.

A sense strand DNA primer as indicated in Sequence No. 11 and an anti-sense strand DNA primer as indicated in Sequence No. 12 were synthesized. Using the synthetic DNAs as the primers and pRH71 as a template, the PCR was conducted to obtain an amplification fragment of 846 bp encoding the structural gene of L-proline-4-hydroxylase. This fragment was cleaved by Eco RI and Hind III, then inserted into the Eco RI-Hind III cleavage site of plasmid pBTac1 containing Ptac (produced by Boehringer Mannheim), and transformed into E. coli XL1-Blue MRF' stain.

Plasmid pTc4OH in which the DNA fragment containing the structural gene of L-proline-4-hydroxylase was inserted in the same direction as the transcription direction of Ptac was obtained from the resulting transformant as shown in Fig. 3.

### (3) Construction of an Expression Plasmid Using Ptrpx2:

In the same manner as in (1) of Example 3, an amplified fragment containing the structural gene of L-proline-4-hydroxylase was recovered and processed with restriction enzymes, and an ethanol precipitate was then obtained by ethanol precipitation. The ethanol precipitate was dissolved in 5 µl of TE.

An ATG vector, pTrS32 formed by combining a synthetic linker and plasmid pKYP200, which is composed of a basic plasmid pBR322 together with two promoters Ptrps connected in series (Ptrpx2), was cleaved with Hind III and Bam HI. Hind III-Bam HI fragment containing the structural gene of L-proline-4-hydroxylase in the above was inserted into the Hind III-Bam HI cleavage site of the vector, using a ligation kit (produced by Takara Shuzo Co.).

Using the thus-obtained plasmid, E. coli XL1-Blue MRF' strain were transformed in a usual manner. The transformant was spread on LB-agar medium containing 50 µg/ml ampicillin and then cultivated thereon overnight at 37°C. The plasmid was extracted from the grown colonies of the transformant cells in a usual manner, and the structure of the plasmid was identified by digestion with restriction enzyme. The part of the structural gene of L-proline-4-hydroxylase was sequenced to determine its nucleotide sequence, using a base sequencing kit (Taq DyeDeoxy™ Terminator Cycle Sequencing Kit, produced by Applied Biosystems Co.), which revealed that the nucleotide sequence of the structural gene is indicated by Sequence No. 2.

As a results, plasmid pTr2-40H in which the DNA fragment encoding the structural gene of L-proline-4-hydroxylase was inserted in the same direction as the transcription direction of Ptrpx2 was obtained shown in Fig. 4.

A DNA as indicated in Sequence No. 13 and a DNA as indicated in Sequence No. 14 were synthesized, using 380A·DNA Synthesizer (produced by Applied Biosystems Co.) . These DNAs were so designed that their 3' terminals of 25 bp are complementary to each other. These DNAs each have a nucleotide sequence coding for the N-terminal site of Dactylosporangium sp. RH1-derived L-proline-4-hydroxylase protein, in which the nucleotide sequence has been site-specifically substituted to make it a codon that is the most suitable in its expression in Escherichia coli.

Using these synthetic DNA's as primers and templates, PCR was conducted. The reaction was conducted, using 20 µl of a reaction mixture comprising 0.5 U of Pfu DNA polymerase (produced by STRATAGENE Co.), 2 µl of x10 buffer for Pfu DNA polymerase, 2 µl of DMSO, 1 µl of 2.5 mM dNTP, 2 µM of the synthetic DNA of Sequence No. 13 and 2 µM of the synthetic DNA of Sequence No. 14. The reaction mixture was incubated at 96°C for 5 minutes. Subsequently, a three step incubation, namely at 96°C for 2 minutes, at 50°C for 1 minute and at 75°C for 1 minute was repeated for a total of 35 times. After the resulting reaction mixture was subjected to 15% polyacrylamide gel electrophoresis, the formation of an amplified fragment of 107 bp was identified. The amplified fragment was recovered from the gel in the same manner as in (3) of Example 1. The both terminals of the thus-recovered DNA fragment of 107 bp were cleaved with Hind III and Sal I, and the thus-processed fragment was recovered using MERmaid Kit (produced by Bio. Inc.) . The amount of the liquid thus recovered was 16 µl.

Plasmid pTr2-40H DNA was cleaved with Bam HI and Pvu II. The reaction mixture was subjected to agarose gel electrophoresis, through which the formation of two fragments was identified. Of these, the longer fragment having the structural gene of L-proline-4-hydroxylase was isolated, using Prep-A-Gene (produced by Biorad Co.), and its terminals were blunted using a blunting kit (produced by Takara Shuzo Co.) and then cyclized using a ligation kit (produced by Takara Shuzo Co.). With the thus-obtained plasmid, E. coli JM109 strain was transformed in a usual manner, and the resulting transformant cells were spread on LB-agar medium containing 50 µg/ml ampicillin and then cultivated thereon overnight at 37°C. A plasmid was extracted from the grown colonies of the transformant cells in a usual manner, and its structure was identified through digestion with restriction enzyme. As a result of the above, obtained was plasmid pTr2-40HΔ, which is lacking for a part of the sequence of pTr2-40H (see Fig. 5).

Plasmid pTr2-4OHΔ DNA was cleaved with Hind III and Sal I. The PCR-amplified fragment that had been processed with Hind III and Sal I in the above was inserted into the Hind III-Sal I cleavage site of the plasmid, using a ligation kit (produced by Takara Shuzo Co.). With the thus-obtained plasmid, cells of E. coli XL1-Blue MRF' strain were transformed in a usual manner, and the resulting transformant cells were spread on LB-agar medium containing 50 µg/ml of ampicillin and then cultivated thereon overnight at 37°C. A plasmid was extracted from the grown colonies of the transformant cells in a usual manner, and its structure was identified through digestion with restriction enzyme. The part of the plasmid into which the PCR-amplified fragment had been inserted was sequenced, using a base sequencing kit (Taq DyeDeoxy™ Terminator Cycle Sequencing Kit, produced by Applied Biosystems Co.), to determine its nucleotide sequence, which revealed that the nucleotide sequence is indicated by Sequence No. 15.

As a result of the above, obtained was plasmid pWFH1 containing the structural gene DNA fragment coding for the amino acid sequence which is entirely the same as the Dactylosporangium sp. RH1-derived L-proline-4-hydroxylase except that from the 5'-terminal to the Sal I site of the structural gene is partly different from the Dactylosporangium sp. RH1-derived nucleotide sequence, in the same direction as transcription direction of Ptrpx2 (see Fig. 6).

### Example 4: Production of L-proline-4-hydroxylase by Transformant:

E. coli ATCC12435 was transformed with plasmids, pTrl4, pTc4OH and pWFH1 as obtained in Example 3 to obtain transformants, E. coli ATCC12435/pTr14, E. coli ATCC12435/pTc4OH and E. coli ATCC12435/pWFH1, respectively. E. coli ATCC12435/pTr14 and E. coli ATCC12435/pTc4OH were separately inoculated each in 3 ml of an LB medium containing 50 µg/ml ampicillin and cultivated therein overnight at 30°C with shaking.

E. coli ATCC12435/pWFH1 was inoculated in 50 ml of a Med4 medium [1 % polypeptone (produced by Nippon Pharmaceuticals Co.), 0.5 % yeast extract (produced by Difco Co.) and 1 of NaCl] containing 100 µg/ml ampicillin and cultivated therein for 16 hours at 30°C. The resulting culture was used as a seed culture, which was inoculated in a 5 liter jar fermenter filled with 2 liters of a Med6 medium (2 % glucose, 1 % ammonium sulfate,. 0.1 % K₂HPO₄, 0.2 % NaCl, 0.05% MgSO₄, 0. 0278% FeSO₄, 0.0015 % CaCl₂, 0.4 % polypeptone), to which was added 200 mM L-proline. The mixture was subjected to the cultivation in the jar fermenter under the condition of 400 rpm and 1 vvm, at 30°C for 48 to 72 hours. During the incubation, glucose and L-proline were suitably added to the medium in such a manner that glucose was always present in the medium and L-proline could be at about 50 mM therein, and the lowermost limit of the pH of the medium was controlled at 6.5 by adding NH₄OH to the medium.

The thus-obtained cultures were centrifuged respectively to separate the cells.

The L-proline-4-hydroxylase activity of the cells was measured under the conditions mentioned below. If desired, the cells can be frozen and stored at -20°C, and the frozen cells can be thawed and used in the measurement of the enzymatic activity.

The cells separated as above were added to 250 µl of a reaction mixture [comprising 12 mM L-proline, 24 mM 2-ketoglutaric acid, 4 mM ferrous sulfate and 8 mM L-ascorbic acid in 240 mM MES buffer (pH 6.5)] in an amount of 4 % (w/v) in terms of the wet cells and reacted at 35°C for 10 minutes. The reaction mixture was heated at 100°C for 2 minutes to stop the reaction.

After the reaction was stopped, the resulting reaction mixture was centrifuged, and 100 µl of 0.3M borate buffer (pH 10.7), 4 µl of 10 % (v/v) mercaptoethanol and 16 µl of 5 % % (w/v) o-phthalaldehyde in ethanol were added to 100 µl of the resulting supernatant and the reaction mixture was kept at 60°C for 30 seconds. Then, 50 µl of 2 % (w/v) NBD in ethanol was added to the reaction mixture and kept at 60°C for 40 minutes. Thirty microliters of 1 N HCl was added to the reaction mixture to stop the reaction. The resulting reaction mixture was centrifuged and filtrated through a filter to remove the precipitate therefrom, and the resulting filtrate was analyzed through HPLC by which the product trans-4-hydroxy-L-proline produced was quantitatively determined.

The HPLC was conducted under the conditions mentioned below.
- Mobile Phase:: 10 mM citric acid (pH 4.0)/methanol = 3/1 (v/v).
- Flow Rate:: 1 ml/min.
- Column:: YMC Pack ODS AQ-312 (produced by YMC Co., 6 x 150 mm).
- Column Temperature:: 50°C.
- Wavelength for Detection:: excited wavelength of 503 nm
emission wavelength of 541 nm.

As is shown in Table 1 below, the transformants produced L-proline-4-hydroxylase by from 210 to 1420 times/cell as much as the Dactylosporangium sp. RH1 strain which had been used as the gene source.

**Table 1**

| L-Proline-4-hvdroxvlase Activity Produced by Transformants | | |
|---|---|---|
| Strain | Cell Activity¹⁾ | Relative Activity²⁾ |
| E. coli ATCC12435/pWFH1 | 40.00 | 1428 |
| E. coli ATCC12435/pWFH1³⁾ | 4.96 | 177 |
| E. coli ATCC12435/pTr14 | 10.68 | 381 |
| E. coli ATCC12435/pTc4OH | 5.98 | 213 |
| E. coli ATCC12435/pTrS30 | Not detected. | - |
| E. coli ATCC12435/pBTac1 | Not detected. | - |
| Dactylosporangium sp. RH1⁴⁾ | 0.028 | 1 |
| Streptomyces griseoviridis JCM4250⁵⁾ | 0.020 | 0.7 |
| Streptomyces daghestanicus JCM4365⁵⁾ | 0.009 | 0.3 |

| | | |
|---|---|---|
| 1) Cell activity indicates the enzymatic activity per mg of wet cells (U/mg wet cells). One U indicates the enzymatic activity of producing 1 nmol of trans-4-hydroxy-L-proline per minute (nmol/min). | | |
| 2) Relative activity is based on the enzymatic activity produced by Dactylosporangium sp. RH1 strain of being 1 (one). | | |
| 3) The strain was cultivated in the same manner as above but in the absence of L-proline in the jar fermenter. | | |
| 4) described in Reference Example 2. | | |
| 5) described in Reference Example 3. | | |

### Example 5: Construction of an expression plasmid for a fused protein:

### (1) Construction of an expression plasmid for a fused protein with a β-galactosidase protein fragment:

After 2.4 µg of plasmid pBluescript II KS(+) DNA was cleaved with Restriction endonucleases Eco RV and XbaI, ethanol precipitate was obtained by the ethanol precipitation. The ethanol precipitate was dissolved in 5 µl of TE.

After 4 µg of plasmid pRH71 DNA was cleaved with Restriction endonuclease Sac I, an ethanol precipitate was obtained in the same manner as mentioned above. After the ethanol precipitate (DNA fragment) was dissolved in 36 µl of TE, both terminals of the DNA fragment were blunted using a Takara DNA Blunting Kit produced by Takara Shuzo Co., Ltd. The treated DNA was subjected to agarose gel electrophoresis. A DNA fragment of approximately 2.4 kb was extracted from the gel in a usual manner, and recovered using a Prep-A-gene produced by Biorad Co. The recovered DNA was cleaved with Xba I, and an ethanol precipitate was obtained in the same manner as mentioned above. The ethanol precipitate (DNA fragment) was dissolved in 10 µl of TE.

The above Eco RV-XbaI cleaved pBluescript IIKS(+) DNA was ligated with the Sac I cleaved, blunted and Xba I cleaved DNA recovered above using Takara Ligation Kit produced by Takara Slurzo Co. Ltd.

After E. coli XL2-Blue MRF' strain (produced by Stratagene) was transformed using the thus-ligated DNA, the transformant was spread on LB agar medium containing 50 µg/ml ampicillin, 0.2 mM IPTG and 40 µg/ml X-Gal, and cultivated overnight at 37°C.

The plasmid was extracted in a usual manner from the colony grown on the medium, and the structure of the plasmid was identified by digestion with restriction endonuclease.

Further, the PCR was conducted using the plasmid as a template, DNA indicated in Sequence No. 16 as a sense strand primer and DNA indicated in Sequence No. 7 as an anti-sense'strand primer. Since a DNA fragment of 50 bp, corresponding to an N-terminal amino acid sequence of L-proline-4-hydroxylase, was formed by the above-mentioned reaction, it was confirmed that the structural gene of the intended L-proline-4-hydroxylase was inserted into the plasmid.

Plasmid pES1-23a in which the structural gene of L-proline-4-hydroxylase was inserted in the same direction as the transcription direction of lac promoter in the form fused with 34 N-terminal amino acids of β-Gal was obtained by the above-mentioned method as shown in Fig. 7. The amino acid sequence of the fused protein formed is shown in Sequence No. 18.

### (2) Construction of an expression plasmid for a fused protein with a maltose binding protein:

Using DNA indicated in Sequence No. 17 as a sense strand primer, DNA indicated in Sequence No. 12 as an antisense strand primer and pRH71 as a template, the PCR was conducted in the same manner as in (3) of Example 1. That is, 20 µl of a reaction mixture containing 0.1 µg of pRH71, 2 µM of the sense strand DNA primer and 2 µM of the anti-sense strand DNA primer was incubated at 96°C for 5 minutes. Subsequently, a three step incubation, namely at 96°C for 2 minutes, at 58°C for 1 minute and at 75°C for 1 minute was repeated for a total of 30 times.

After the reaction mixture was subjected to agarose gel electrophoresis, an amplified fragment of 833 bp containing a structural gene of L-proline-4-hydroxylase was extracted in a usual manner, and the DNA fragment was recovered using a Prep-A-gene produced by Biorad Co. The DNA fragment of 833 bp recovered was cleaved with Hind III, and an ethanol precipitate was then obtained by the ethanol precipitation. The ethanol precipitate was dissolved in 5 µl of TE, and used as the structural gene fragment of L-proline-4-hydroxylase.

Plasmid pMAL-c2 having only a structural gene of a maltose binding protein without a signal sequence (Protein Fusion & Purification System produced by New England Biolabs) was cleaved with Xmn I and Hind III while regulating by Ptac.

The structural gene fragment of L-proline-4-hydroxylase was inserted into the Xmn I-Hind III cleavage site of pMAL-c2 using a DNA ligation kit produced by Takara Shuzo Co., Ltd., and E. coli XL2-Blue MRF' strain was transformed in a usual manner. The transformant was spread on an LB agar medium containing 50 µg/ml of ampicillin, and then cultivated overnight at 37°C. The plasmid was extracted from the thus-obtained colony in a usual manner, and the structure of the plasmid was identified by digestion with restriction endonuclease.

Plasmid pMc4OH in which the DNA fragment encoding the structural gene of L-proline-4-hydroxylase was inserted in the form fused with the structural gene of the maltose-binding protein under regulation by Ptac was obtained by the above-mentioned method as shown in Fig. 8. The amino acid sequence of the fused protein formed is shown in Sequence No. 19.

### Example 6: Production of L-proline-4-hydroxylase by a transformant containing a fused protein expression plasmid:

E. coli DH1 was transformed using the plasmids pES1-23a and pMc4OH obtained in Example 5. The obtained transformant was cultivated in the same manner as in Example 4 except that a medium containing 0.1 mM IPTG was used, and the productivity of L-proline-4-hydroxylase of the transformant was measured in the same manner as in Example 4.

As shown in Table 2, the transformant produced L-proline-4-hydroxylase in an amount of from 29 to 298 times per cell in comparison to Dactylosporangium sp. RH1 strain used as a gene source.

**Table 2**

| Activities of L-Proline-4-hydroxvlase Produced by Transformants | | |
|---|---|---|
| Strain | Cell Activity¹⁾ | Relative Activity³⁾ |
| E. coli DH1/pES1-23a | 0.80 | 29 |
| E. coli DH1/pMc40H | 8.35 | 298 |
| E. coli DH1/pBluescript IIKS(+) | Not detected | - |
| E. coli DH1/pMAL-c2 | Not detected | - |
| Dactylosporangium sp. RH1²⁾ | 0.028 | 1 |

| | | |
|---|---|---|
| 1) The cell activity is shown in terms of enzymatic activity per mg of wet cells (U/mg-wet cells). One U indicates the enzymatic activity of producing 1 nmol of trans-4-hydroxy-L-proline per minute (nmol/min). | | |
| 2) described in Reference Example 2. | | |
| 3) The relative activity is shown by defining the enzymatic activity given from Dactylosporangium sp. RH1 strain as 1. | | |

### Example 7: Production of Trans-4-hydroxy-L-proline by Transformant:

### (1) Production of Trans-4-hydroxy-L-proline by Transformant E. coli ATCC12435/pTr14:

Transformant cells of E. coli ATCC12435/pTr14 as obtained in Example 4 were inoculated in 3 ml of an LB medium containing 100 µg/ml ampicillin and cultivated therein at 30°C for 16 hours with shaking. The resulting culture was centrifuged, and the amount of trans-4-hydroxy-L-proline in the supernatant thus separated was quantitatively determined.

As a result, 381 µM (50.0 mg/liter) trans-4-hydroxy-L-proline was formed in the supernatant of the culture of E. coli ATCC12435/pTr14.

On the other hand, trans-4-hydroxy-L-proline was not detected in the supernatant of the culture of E. coli ATCC12435 which had been used as the host.

### (2) Production of Trans-4-hydroxy-L-proline by Transformant E. coli ATCC12435/pWFH1:

Transformant cells of E. coli ATCC12435/pWFH1 were inoculated in 50 ml of a Med4 medium containing 100 µg/ml ampicillin and 2 % glucose, and cultivated therein at 30°C for 16 hours with shaking. The culture was used as a seed culture. The seed culture was inoculated in a 5 liter jar fermenter filled with 2 liters of a Med6 medium containing 0.8 % peptone in place of polypeptone and the cells were cultivated in the fermenter under the condition of 400 rpm and 1 vvm, at 33°C.

During the cultivation, glucose was suitably added to the medium so as not to make glucose absent in the medium, and the lowermost limit of the pH of the medium was controlled at 6.5 by adding NH₄OH to the medium.

The culture was centrifuged, and the amount of trans-4-hydroxy-L-proline in the supernatant separated was quantitatively determined. Fifty two hours after the start of the incubation, 10.7 mM (1.4 g/liter) trans-4-hydroxy-L-proline was produced and accumulated in the supernatant of the culture of E. coli ATCC12435/pWFH1.

On the other hand, free trans-4-hydroxy-L-proline was not detected in the supernatant of the culture of E. coli ATCC12435 used as the host.

### (3) Production of Trans-4-hydroxy-L-proline by Transformant E. coli ATCC12435/pWFH1:

Transformant cells of E. coli ATCC12435/pWFH1 were inoculated in 50 ml of a Med4 medium containing 100 µg/ml ampicillin and cultivated therein at 30°C for 16 hours with shaking. The culture was used as a seed culture. The seed culture was inoculated in a 5 liter jar fermenter filled with 2 liters of a Med6 medium. 200mM L-proline was added to the medium. The cells were cultivated in the fermenter under the condition of 400 rpm and 1 vvm, at 30°C.

During the cultivation, glucose and L-proline were suitably added to the medium in such a manner that glucose was always present in the medium and that L-proline could be at about 50 mM therein, and the lowermost limit of the pH of the medium was controlled at 6.5 by adding NH₄OH to the medium.

The culture was centrifuged, and the amount of trans-4-hydroxy-L-proline in the supernatant separated was quantitatively determined. Seventy two hours after the start of the incubation, 185 mM (24 g/liter) trans-4-hydroxy-L-proline was produced and accumulated in the supernatant of the culture of E. coli ATCC12435/pWFH1.

On the other hand, free trans-4-hydroxy-L-proline was not detected in the supernatant of the culture of E. coli ATCC12435 used as the host.

### (4) Production of Trans-4-hydroxy-L-proline by Transformant E. coli ATCC12435/pMc4OH:

Transformant cells of E. coli ATCC12435/pMc4OH were inoculated in 50 ml of a Med4 medium containing 100 µg/ml ampicillin and cultivated therein at 30°C for 16 hours with shaking. The culture was used as a seed culture. The seed culture was inoculated in a 5 liter jar fermenter filled with 2 liters of a Med6 medium. 200 mM L-proline was added to the medium. The cells were cultivated in the fermenter under the condition of 400 rpm and 1vvm, at 30°C.

During the incubation, glucose and L-proline were suitably added to the medium in such a manner that glucose was always present in the medium and that L-proline could be at about 50 mM therein, and the lowermost limit of the pH of the medium was controlled at 6.5 by adding NH₄OH to the medium.

The culture was centrifuged, and the amount of trans-4-hydroxy-L-proline in the supernatant separated was quantitatively determined. Seventy two hours after the start of the incubation, 85.4 mM (11.2 g/liter) trans-4-hydroxy-L-proline was produced and accumulated in the supernatant of the culture of E. coli ATCC12435/pMc4OH.

On the other hand, free trans-4-hydroxy-L-proline was not detected in the supernatant of the culture of E. coli ATCC12435 which had been used as the host.

### Example 8: Conversion of L-proline into Trans-4-hydroxy-L-proline with Transformant cells:

Transformant cells of E. coli ATCC12435/pTr14 were inoculated in 10 ml of an LB medium containing 50 µg/ml ampicillin and cultivated therein overnight at 30°C with shaking. The culture was centrifuged to collect the cells. If desired, the cells were frozen and stored at -20°C and thawed before use.

The cells were added to 250 µl of a reaction mixture (comprising 20 mM L-proline, 24 mM 2-ketoglutaric acid, 4 mM ferrous sulfate and 8 mM L-ascorbic acid in 240 mM MES buffer, pH 6.5) at 10 % (w/v) in terms of the wet cells, and reacted at 35°C for 60 minutes. The amount of trans-4-hydroxy-L-proline as formed in the reaction mixture was quantitatively determined. In the mixture, 11.5 mM (1.5 g/liter) trans-4-hydroxy-L-proline was produced.

### Reference Example 1: Isolation and Purification of L-proline-4-hydroxylase:

### (1) Preparation of Frozen Cells of Dactylosporangium sp. RH1:

SR3 medium (comprising 1.0 % glucose, 1.0 % soluble starch, 0.5 % yeast extract, 0.5 % tryptone, 0.3 % meat extract and 0.05 % magnesium phosphate and adjusted at pH 7.2 with 6 N NaOH) was put into a 2-liter Erlenmeyer flask in an amount of 200 ml and sterilized at 120°C for 20 minutes. Cells of Dactylosporangium sp. RH1 that had grown in an HT-agar plate medium (comprising 1 % soluble starch, 0.2 % NZ-amine, 0.1 % yeast extract, 0.1 % meat extract and 1.5 % agar, adjusted at pH 7.2 with 6 N NaOH, and sterilized at 120°C for 20 minutes) were inoculated in the SR3 medium and cultivated at 28°C for 2 days with shaking. The resulting culture was used as a seed culture in the following steps.

Two liter of Df1 medium (comprising 5 % soluble starch, 1.5 % soybean meal, 0.05% monopotassium phosphate, 0.05 % magnesium sulfate 7-hydrate and 0.5 % calcium carbonate, and adjusted at pH 7.0 with 6 N NaOH) was put into a 5-liter jar fermenter and sterilized at 120°c for 20 minutes. The seed culture prepared above was inoculated into the medium under germ-free conditions and cultivated therein at 700 rpm, at 1 vvm and at 28°C for 2 days. The pH of the medium was not controlled during the cultivation. The resulting culture was centrifuged at 7,000 x g for 10 minutes at 4°C. Thus 75 g of wet cells per liter of the culture was obtained. The wet cells were washed with physiological saline solution at 4°C, centrifuged, then frozen and stored at -80°C until use.

### (2) Preparation of Cell-free Extract:

Six hundred grams of the wet cells obtained in (1) of Reference Example 1 was thawed and suspended in 3 liters of Buffer A [50 mM TAPS buffer (pH 9.0) containing 2 mM DTT, 0.2 mM EDTA and 20% (v/v) glycerol] while cooling with ice. The resulting suspension was milled by a Dyno-mill (produced by Willy A Bachofen Maschinenfabrik, Basel, Switzerland) to disrupt the cells. The thus-disruped cell suspension was subjected to centrifugation at 6,500 x g at 4°C for 30 minutes to separate the supernatant.

The subsequent operations were conducted under cooling with ice at a temperature of 4°C or lower.

### (3) Isolation and purification by Column Chromatography

### (3)-1 STREAMLINE:

The supernatant obtained in the previous step was passed through a STREAMLINE™ (produced by Pharamacia Co.) filled with 300 ml of DEAE adsorbent that had been equilibrated with Buffer A, whereupon a fraction containing the L-proline-4-hydroxylase was eluted with Buffer A containing 0.3M NaCl.

### (3)-2 DEAE Sepharose Column Chromatography:

The active fraction obtained in the previous step was diluted three times with Buffer A and passed through a DEAE Sepharose column (5 cm x 15 cm) that had been equilibrated with Buffer A. The column was washed with Buffer A, and the fraction containing the enzyme was eluted with Buffer A having a linear concentration gradient of NaCl of 0 to 0.3M.

### (3)-3 Butyl Sepharose Column Chromatography:

NaCl was added to the active fraction obtained in the previous step until an NaCl concentration was 3M. The mixture was passed through a butyl Sepharose column (Butyl Sepharose 4 Fast Flow, 2.6 cm x 13 cm) that had been equilibrated with Buffer A containing 3M NaCl. The enzyme was stepwise eluted with four kinds of buffers each having a different NaCl concentration, Buffer A containing 3M NaCl, Buffer A containing 1.98M NaCl, Buffer A containing 0.99M NaCl and Buffer A containing no NaCl, in this order from the eluent buffer having a higher NaCl concentration to that having a lower NaCl concentration.

### (3)-4 Phenyl.Sepharose Column Chromatography:

NaCl was added to the active fraction obtained in the previous step until the concentration of NaCl was 3M. The mixture was passed through a phenyl Sepharose column (Phenyl Sepharose HP Hiload 16/10, 1.6 cm x 10 cm) that had been equilibrated with Buffer A containing 3M NaCl. The column was washed with Buffer A containing 3M NaCl, and the fraction containing the enzyme was eluted with Buffer A.

### (3)-5 Dye Affinity Column Chromatography:

The active fraction obtained in the previous step was de-salted, using a PD-10 column (produced by Pharmacia Co.), and the resulting fraction was passed through a Reactive Red 120 column (1 cm x 12.7 cm; produced by Sigma Co.) that had been equilibrated with Buffer A. The column was washed with Buffer A, and the fraction containing the enzyme was eluted with Buffer A having a linear concentration gradient of NaCl of 0 to 1.5M.

### (3)-6 Resource Q Column Chromatography:

The active fraction obtained in the previous step was de-salted, using a PD-10 column (produced by Pharmacia Co.) that had been equilibrated with Buffer B [50 mM TAPS buffer (pH 8.0) containing 2 mM DTT, 0.1% (v/v) Tween 20 and 20% (v/v) glycerol], and the resulting fraction was passed through a RESOURCE^{TH} Q column (1 ml; produced by Pharmacia Co.) that had been equilibrated with Buffer B. The fraction containing the enzyme was eluted with Buffer B having a liner concentration gradient of NaCl of 0 to 0.2M.

Summaries of the isolation and purification of the L-proline-4-hydroxylase are shown in Table 3.

**Table 3**

| Summaries of Isolation and Purification of L-proline-4-hydroxylase | | | | |
|---|---|---|---|---|
| Fraction | Total Protein (mg) | Total Activity (U) | Relative Activity (U/mg of protein) | Yield (%) |
| Cell-free Extract | 13,330 | 11,000 | 0.83 | 100 |
| STREAMLINE | 4,875 | 4,880 | 1.00 | 44.4 |
| DEAE Sepharose | 353 | 3,820 | 10.8 | 34.7 |
| Butyl Sepharose | 35.1 | 1,310 | 37.3 | 11.9 |
| Phenyl Sepharose | 1.44 | 814 | 565.3 | 7.4 |
| Color Affinity | 0.212 | 366 | 1,726 | 3.3 |
| Resource Q | 0.100 | 384 | 3,840 | 3.5 |

### Reference Example 2: Production of L-proline-4-hydroxylase by Dactylosporangium sp. RH1:

SR3 medium was put in test tubes in an amount of 10 ml each and sterilized at 120°C for 20 minutes. One loopful of cells of Dactylosporangium sp. RH1, that had grown in HT-agar plate medium , was inoculated into the above-mentioned SR3 medium in each test tube, cultivated at 28°C for 2 days with shaking. The resulting culture was used as a seed culture in the following steps.

Separately, Df1 medium was put in test tubes in an amount of 10 ml each and sterilized at 120°C for 20 minutes. One ml of the above-mentioned seed culture was inoculated in the medium in each test tube under germ-free condition and cultivated at 28°C for 2 days with shaking. The thus-obtained culture was centrifuged at 8000 rpm for 10 minutes at 4°C. The cells thus separated were washed with 80 mM TES [N-tris (hydroxymethyl)methyl-2-aminoethanesulfonic acid] buffer (pH 7.5) arid then recentrifuged. 150 mg of the thus-obtained wet cells was suspended in 1.5 ml of a reaction mixture which had been prepared by adding 1.4% (v/v) Nymeen solution [prepared by adding 4g of Nymeen S-215 (produced by Nippon Oils & Fats Co.) to 10 ml of xylene] to 80 mM TES buffer (pH 7.5) containing 4 mM L-proline, 8 mM α-ketoglutaric acid, 4 mM L-ascorbic acid and 2 mM ferrous sulfate] and the mixture was allowed to stand at 30°C for 30 minuets to carry out the enzymatic reaction.

After the reaction, the cells were removed from the reaction mixture by centrifugation, and the amount of hydroxyproline formed in the supernatant was quantitatively determined, from which the L-proline 4-hydroxylase activity of the cells of Dactylosporangium sp. was determined.

The results are shown in Table 1.

### Reference Example 3: Production of L-proline-4-hydroxylase by Streptomyces griseoviridis

The L-proline-4-hydroxylase activity of Streptomyces griseoviridis JCM4250 and Streptomyces daghestanicus JCM4365 was determined in the same manner as in Reference Example 2. In this example, however, Df4 medium [comprising 2.5 % glycerol, 2.5 % of glucose, 1.5% soybean meal, 0.005% monopotassium phosphate, 0.05 % magnesium sulfate 7-hydrate and 0.5 % calcium carbonate and adjusted to pH 7.0 with 6 N NaOH] was used in place of the Df1 medium.

The results are shown in Table 1.

### INDUSTRIAL APPLICABILITY OF THE INVENTION

According to the present invention, there are provided industrial methods for producing trans-4-hydroxy-L-proline which is useful as a starting compound for medicines and an additive to foods, genes which code for a protein having the,enzymatic activity of hydroxylating the 4-position of L-proline and which are useful for the above-mentioned process, transformants containing the genes, and methods for producing L-proline 4-hydroxylases using the transformants.

### SEQUENCE LISTING

### GENERAL INFORMATION:

### APPLICANT:

NAME: Kyowa Hakko Kogyo Co., Ltd.
STREET: 6-1, Ohtemachi 1-chome, Chiyoda-ku
CITY: Tokyo
COUNTRY: Japan
ZIP: 100

TITLE OF INVENTION: Process for producing trans-4-hydroxy-L-proline

NUMBER OF SEQUENCES: 19

COMPUTER READABLE FORM:
- MEDIUM TYPE:: 3.5" Diskette, 1.44 Mb
- COMPUTER:: IBM PS/2 Model 50Z or 55SX
- OPERATING SYSTEM:: MS-DOS (Version 5.0)
- SOFTWARE:: Microsoft Word

### CURRENT APPLICATION DATA:

APPLICATION NUMBER: EP 96 905 024.4
FILING DATE:

### PRIOR APPLICATION DATA:

APPLICATION NUMBER: PCT/JP9600559
FILING DATE: March 7, 1996

### INFORMATION FOR SEQ ID NO: 1:

SEQUENCE CHARACTERISTICS:
LENGTH: 272 amino acids
TYPE: amino acid
TOPOLOGY: linear
MOLECULE TYPE: protein
ORIGINAL SOURCE:
ORGANISM: Dactylosporangium sp.
STRAIN: RH1

SEQUENCE DESCRIPTION: SEQ ID NO: 1:

Met Leu Thr Pro Thr Glu Leu Lys Gln Tyr Arg Glu Ala Gly Tyr Leu
1               5                   10                  15
Leu Ile Glu Asp Gly Leu Gly Pro Arg Glu Val Asp Cys Leu Arg Arg
            20                  25                  30
Ala Ala Ala Ala Leu Tyr Ala Gln Asp Ser Pro Asp Arg Thr Leu Glu
        35                  40                  45
Lys Asp Gly Arg Thr Val Arg Ala Val His Gly Cys His Arg Arg Asp
    50                  55                  60
Pro Val Cys Arg Asp Leu Val Arg His Pro Arg Leu Leu Gly Pro Ala

65                  70                  75                  80
Met Gln Ile Leu Ser Gly Asp Val Tyr Val His Gln Phe Lys Ile Asn
                85                  90                  95
Ala Lys Ala Pro Met Thr Gly Asp Val Trp Pro Trp His Gln Asp Tyr
            100                 105                 110
Ile Phe Trp Ala Arg Glu Asp Gly Met Asp Arg Pro His Val Val Asn
        115                 120                 125
Val Ala Val Leu Leu Asp Glu Ala Thr His Leu Asn Gly Pro Leu Leu
    130                 135                 140
Phe Val Pro Gly Thr His Glu Leu Gly Leu Ile Asp Val Glu Arg Arg
145                 150                 155                 160
Ala Pro Ala Gly Asp Gly Asp Ala Gln Trp Leu Pro Gln Leu Ser Ala
                165                 170                 175
Asp Leu Asp Tyr Ala Ile Asp Ala Asp Leu Leu Ala Arg Leu Thr Ala
            180                 185                 190
Gly Arg Gly Ile Glu Ser Ala Thr Gly Pro Ala Gly Ser Ile Leu Leu
        195                 200                 205
Phe Asp Ser Arg Ile Val His Gly Ser Gly Thr Asn Met Ser Pro His
    210                 215                 220
Pro Arg Gly Val Val Leu Val Thr Tyr Asn Arg Thr Asp Asn Ala Leu
225                 230                 235                 240
Pro Ala Gln Ala Ala Pro Arg Pro Glu Phe Leu Ala Ala Arg Asp Ala
                245                 250                 255
Thr Pro Leu Val Pro Leu Pro Ala Gly Phe Ala Leu Ala Gln Pro Val
            260                 265                 270

INFORMATION FOR SEQ ID NO: 2:
SEQUENCE CHARACTERISTICS:
LENGTH: 816 base pairs
TYPE: nucleic acid
STRANDEDNESS: double
TOPOLOGY: linear
MOLECULE TYPE: genomic DNA
ORIGINAL SOURCE:
ORGANISM: Dactylosporangium sp.
STRAIN: RH1
FEATURE:
IDENTIFICATION METHOD: by experiment

SEQUENCE DESCRIPTION: SEQ ID NO: 2:

ATGCTGACCC CGACGGAGCT CAAGCAGTAC CGCGAGGCGG GCTATCTGCT CATCGAGGAC 60
GGCCTCGGCC CGCGGGAGGT CGACTGCCTG CGCCGGGCGG CGGCGGCCCT CTACGCGCAG 120
GACTCGCCGG ACCGCACGCT GGAGAAGGAC GGCCGCACCG TGCGCGCGGT CCACGGCTGC 180
CACCGGCGCG ACCCGGTCTG CCGCGACCTG GTCCGCCACC CGCGCCTGCT GGGCCCGGCG 240
ATGCAGATCC TGTCCGGCGA CGTGTACGTC CACCAGTTCA AGATCAACGC GAAGGCCCCG 300
ATGACCGGCG ATGTCTGGCC GTGGCACCAG GACTACATCT TCTGGGCCCG AGAGGACGGC 360
ATGGACCGTC CGCACGTGGT CAACGTCGCG GTCCTGCTCG ACGAGGCCAC CCACCTCAAC 420
GGGCCGCTGT TGTTCGTGCC GGGCACCCAC GAGCTGGGCC TCATCGACGT GGAGCGCCGC 480
GCGCCGGCCG GCGACGGCGA CGCGCAGTGG CTGCCGCAGC TCAGCGCCGA CCTCGACTAC 540
GCCATCGACG CCGACCTGCT GGCCCGGCTG ACGGCCGGGC GGGGCATCGA GTCGGCCACC 600
GGCCCGGCGG GCTCGATCCT GCTGTTCGAC TCCCGGATCG TGCACGGCTC GGGCACGAAC 660
ATGTCGCCGC ACCCGCGCGG CGTCGTCCTG GTCACCTACA ACCGCACCGA CAACGCCCTG 720

CCGGCGCAGG CCGCTCCGCG CCCGGAGTTC CTGGCCGCCC GCGACGCCAC CCCGCTGGTG 780
CCGCTGCCCG CGGGCTTCGC GCTGGCCCAG CCCGTC 816

INFORMATION FOR SEQ ID NO: 3:
SEQUENCE CHARACTERISTICS:
LENGTH: 17 base pairs
TYPE: nucleic acid
STRANDEDNESS: single
TOPOLOGY: linear
MOLECULE TYPE: other nucleic acid, synthetic DNA

SEQUENCE DESCRIPTION: SEQ ID NO: 3:

ATGCTSACSC CNACNGA 17

INFORMATION FOR SEQ ID NO: 4:
SEQUENCE CHARACTERISTICS:
LENGTH: 17 base pairs
TYPE: nucleic acid
STRANDEDNESS: single
TOPOLOGY: linear
MOLECULE TYPE: other nucleic acid, synthetic DNA

SEQUENCE DESCRIPTION: SEQ ID NO: 4:

GGSCCSAGNC CRTCYTC 17

INFORMATION FOR SEQ ID NO: 5:
SEQUENCE CHARACTERISTICS:
LENGTH: 71 base pairs
TYPE: nucleic acid
STRANDEDNESS: double
MOLECULE TYPE: other nucleic acid, synthetic DNA

SEQUENCE DESCRIPTION: SEQ ID NO: 5:

ATG CTG ACG CCG ACG GAG CTC AAG CAG TAC CGC GAG GCG GGC TAT CTG 48
Met Leu Thr Pro Thr Glu Leu Lys Gln Tyr Arg Glu Ala Gly Tyr Leu
1               5                   10                  15
CTC ATC GAG GAC GGT CTG GGC CC 71
Leu Ile Glu Asp Gly Leu Gly
            20

INFORMATION FOR SEQ ID NO: 6:
SEQUENCE CHARACTERISTICS:
LENGTH: 20 base pairs
TYPE: nucleic acid
STRANDEDNESS: single
TOPOLOGY: linear
MOLECULE TYPE: other nucleic acid, synthetic DNA
SEQUENCE DESCRIPTION: SEQ ID NO: 6:

ACGGAGCTCA AGCAGTACCG 20

INFORMATION FOR SEQ ID NO: 7:
SEQUENCE CHARACTERISTICS:
LENGTH: 19 base pairs
TYPE: nucleic acid
STRANDEDNESS: single
TOPOLOGY: linear
MOLECULE TYPE: other nucleic acid, synthetic DNA

SEQUENCE DESCRIPTION: SEQ ID NO: 7:

GGGCCGAGAC CGTCCTCGA 19

INFORMATION FOR SEQ ID NO: 8:
SEQUENCE CHARACTERISTICS:
LENGTH: 2707 base pairs
TYPE: nucleic acid
STRANDEDNESS: double
TOPOLOGY: linear
MOLECULE TYPE: genomic DNA
ORIGINAL SOURCE:
ORGANISM: Dactylosporangium sp.
STRAIN: RH1

SEQUENCE DESCRIPTION: SEQ ID NO: 8:

GAGCTCTACC GGCGAACGCG CNCNCGGTGG CCGAATACGA NCCGGCGCCC CACGATGTNC 60
GGGCCACCCT CGTGCAGNCG GCCGAGCAGG ACGCCGGGCT ACGGGCGGCG NCGGTCGAGN 120
CGTGGACCCG CGCCTGCGGG GCGCCCCCGN CGGTGCATGT GCTGCCGGGC GGGCACTTCT 180
CGCTCTGCGC CGGCCGCACG TCGAGCGGCT GGCCCGGCTC CTGCCCGGCC TGTAGGCGAC 240
CTAACCCACC GTGAGGAGCG CTCATGCTGA CCCCGACGGA GCTCAAGCAG TACCGCGAGG 300
CGGGCTATCT GCTCATCGAG GACGGCCTCG GCCCGCGGGA GGTCGACTGC CTGCGCCGGG 360
CGGCGGCGGC CCTCTACGCG CAGGACTCGC CGGACCGCAC GCTGGAGAAG GACGGCCGCA 420
CCGTGCGCGC GGTCCACGGC TGCCACCGGC GCGACCCGGT CTGCCGCGAC CTGGTCCGCC 480
ACCCGCGCCT GCTGGGCCCG GCGATGCAGA TCCTGTCCGG CGACGTGTAC GTCCACCAGT 540
TCAAGATCAA CGCGAAGGCC CCGATGACCG GCGATGTCTG GCCGTGGCAC CAGGACTACA 600
TCTTCTGGGC CCGAGAGGAC GGCATGGACC GTCCGCACGT GGTCAACGTC GCGGTCCTGC 660
TCGACGAGGC CACCCACCTC AACGGGCCGC TGTTGTTCGT GCCGGGCACC CACGAGCTGG 720
GCCTCATCGA CGTGGAGCGC CGCGCGCCGG CCGGCGACGG CGACGCGCAG TGGCTGCCGC 780
AGCTCAGCGC CGACCTCGAC TACGCCATCG ACGCCGACCT GCTGGCCCGG CTGACGGCCG 840
GGCGGGGCAT CGAGTCGGCC ACCGGCCCGG CGGGCTCGAT CCTGCTGTTC GACTCCCGGA 900
TCGTGCACGG CTCGGGCACG AACATGTCGC CGCACCCGCG CGGCGTCGTC CTGGTCACCT 960
ACAACCGCAC CGACAACGCC CTGCCGGCGC AGGCCGCTCC GCGCCCGGAG TTCCTGGCCG 1020
CCCGCGACGC CACCCCGCTG GTGCCGCTGC CCGCGGGCTT CGCGCTGGCC CAGCCCGTCT 1080
AGGCTGCCGC AGGCGGCGCA CGGCCCACCT CAGCGCAGGC CGAGCAGCCG CCCCACGCCG 1140
GCGGCGAAGC GGATCAGGCC GCGCAGGCCG AGCGGCGGGC GAGGCGAGCA TTGTCGGGCT 1200
GCCCAGTCGT CGTGTCGTGG GTGACAGCCC GTGGCGGCTC TTCTGACGGC CCGCGACGGA 1260
TCACGGTCAT GTTTGCCGAT GGGACGTCAC GGTCGTGTGC CCGGACGGTC GAAAATCACC 1320
AGAATGGTGC TGATGGCCTG TCGCGGGGTG TCGGTGGTGC GGTCGCCAGC ATCCCTCGGC 1380
CGCGCCGGGG CCGGTGCGTC TCACCAGGCC GTGCGGGCGT TGACGGCCGA GACGGCGGAC 1440
CGGAAGGCGG CCACCGGTTC GCCGAGATAG CACCACGGGA ACTCGGTGAC CAACTCGCCG 1500
ATGGCCAGAA ACTGCATCGC CGCCGCCTCG TCGTCGCCGG CCGCCAGAAA CGCGACCGCG 1560
AACGCGTTGG CGTCCAGGGC CCAGTCCCGG CGCCGCACAT AGCGGCGGTG CCGGACCGAC 1620
TCGCGGGCCG CGTCGTTGAG CGACTCCCGC ACCGCCGCCG AGCGGATGTA GTCACGGCTA 1680
GGCGCGCCAC CGAGGTCCAG CCAGTGTTCA AGGTGCGCCA GGGCGACGAG CGTGCCAAGA 1740

CGGCTGCCCT CGCCTGCGTC CCGCGCGGCC CCCTCGGCGA ACGCGCGCAT CCGCTCCGGC 1800
GAGCCGCCCC ACTTCCGGCA GAGGTTCTGC AGCATCGCGG TGTGGGCGCG GTAGTTGTCC 1860
GGGTCATGCG CGACCGCCGC ATCGAACCGG CGCCGCTGCT CGTCCGCACT CAGACCCAGG 1920
CCACGGGCGA CGGTGATCAG GCCAGTCCAG GCCGTGACAT TGGCCGGCTC CCGGCGTACC 1980
ACCTCCTGCA GGCAGTACTC GGCGAGTTCC AGCCGCTGCC GGAACAGCAC CCACGCTCAG 2040
GCGGGACGTA CGCGGCAGGA AGCCCGGTGC GAGCCTCCCA GGCCCACGCG ATCGCCCGGT 2100
GACCGCGCAC GAGCAGCGCC AGGGGATCGT CCGGCCCGTG CTCGATTACA TGCGCACAGC 2160
ACAGTGCTCG GTGCCGGGGA CCTGGGCCGC GACCGAGACC AGGAAGTCGA GATCCTCTCT 2220
TGTGCCGCTC GGCGGCCAGN ATGGGCCGCG CGGNAAGCCA GTCTCCGGCG GCCAACGCTG 2280
CCCACAGCCA CCGGGCGTCG GCATCACCGA GCGTCGGGTC GAAGGCGCGC GCCACGCGCC 2340
CTGCGGACCG CCGGAACAGG GGCATGCGCG CATCCTCCAG CCGATGCGCC GATCAGCCGG 2400
CGCGGCAAGA TCGTACGCCC GGACCGCGAG GTCGGGAGGT CCACGGGCGG TCCCCACTGG 2460
GCGACGACTG TCAGNTGCTA CGCTGGCCCG GTGGCCGAGA TCACCGGGGC GTTCGAGATC 2520
CATGTAACCG TCGAGGCGCA CCACGGCACG GACCTCGCCC GGTTCGCCGA GAAGCACGAC 2580
GTCAAGTTCC TGCACATCGT CCTGGACCGC GGCCGGTTTC CGTCCCAGCC GATCTCACGC 2640
TGCCGATGCA CGGCACCCTC GCTCAGGCAC GGAAGACGGC GCCACGTGGC GGGAGCGGCT 2700
ACTCGAG 2707

INFORMATION FOR SEQ ID NO: 9:
SEQUENCE CHARACTERISTICS:
LENGTH: 37 base pairs
TYPE: nucleic acid
STRANDEDNESS: single
TOPOLOGY: linear
MOLECULE TYPE: other nucleic acid, synthetic DNA

SEQUENCE DESCRIPTION: SEQ ID NO: 9:

GTGAGGAAAG CTTATGCTGA CCCCGACGGA GCTCAAG 37

INFORMATION FOR SEQ ID NO: 10:
SEQUENCE CHARACTERISTICS:
LENGTH: 36 base pairs
TYPE: nucleic acid
STRANDEDNESS: single
TOPOLOGY: linear
MOLECULE TYPE: other nucleic acid, synthetic DNA

SEQUENCE DESCRIPTION: SEQ ID NO: 10:

GCCTGCGGGA TCCTAGACGG GCTGGGCCAG CGCGAA 36

INFORMATION FOR SEQ ID NO: 11:
SEQUENCE CHARACTERISTICS:
LENGTH: 37 base pairs
TYPE: nucleic acid
STRANDEDNESS: single
TOPOLOGY: linear
MOLECULE TYPE: other nucleic acid, synthetic DNA

SEQUENCE DESCRIPTION: SEQ ID NO: 11:

GTGAGGAGAA TTCATGCTGA CCCCGACGGA GCTCAAG 37

INFORMATION FOR SEQ ID NO: 12:
SEQUENCE CHARACTERISTICS:
LENGTH: 38 base pairs
TYPE: nucleic acid
STRANDEDNESS: single
TOPOLOGY: linear
MOLECULE TYPE: other nucleic acid, synthetic DNA

SEQUENCE DESCRIPTION: SEQ ID NO: 12:

CCGCCTGAAG CTTCCTAGAC GGGCTGGGCC AGCGCGAA 38

INFORMATION FOR SEQ ID NO: 13:
SEQUENCE CHARACTERISTICS:
LENGTH: 64 base pairs
TYPE: nucleic acid
STRANDEDNESS: single
TOPOLOGY: linear
MOLECULE TYPE: other nucleic acid, synthetic DNA

SEQUENCE DESCRIPTION: SEQ ID NO: 13:

TGAGGAAAGC TTATGCTGAC CCCGACCGAA CTGAAACAGT ATCGTGAAGC 50
GGGCTATCTG CTGA 64

INFORMATION FOR SEQ ID NO: 14:
SEQUENCE CHARACTERISTICS:
LENGTH: 66 base pairs
TYPE: nucleic acid
STRANDEDNESS: single
TOPOLOGY: linear
MOLECULE TYPE: other nucleic acid, synthetic DNA

SEQUENCE DESCRIPTION: SEQ ID NO: 14:

CCGGAATTCG TCGACTTCAC GCGGGCCCAG GCCATCTTCA ATCAGCAGAT 50
AGCCCGCTTC ACGATA 66

INFORMATION FOR SEQ ID NO: 15:
SEQUENCE CHARACTERISTICS:
LENGTH: 816 base pairs
TYPE: nucleic acid
STRANDEDNESS: double
TOPOLOGY: linear
MOLECULE TYPE: other nucleic acid

SEQUENCE DESCRIPTION: SEQ ID NO: 15:

ATG CTG ACC CCG ACC GAA CTG AAA CAG TAT CGT GAA GCG GGC TAT CTG 48
Met Leu Thr Pro Thr Glu Leu Lys Gln Tyr Arg Glu Ala Gly Tyr Leu
1               5                   10                  15
CTG ATT GAA GAT GGC CTG GGC CCG CGT GAA GTC GAC TGC CTG CGC CGG 96
Leu Ile Glu Asp Gly Leu Gly Pro Arg Glu Val Asp Cys Leu Arg Arg

            20                  25                  30
GCG GCG GCG GCC CTC TAC GCG CAG GAC TCG CCG GAC CGC ACG CTG GAG 144
Ala Ala Ala Ala Leu Tyr Ala Gln Asp Ser Pro Asp Arg Thr Leu Glu
        35                  40                  45
AAG GAC GGC CGC ACC GTG CGC GCG GTC CAC GGC TGC CAC CGG CGC GAC 192
Lys Asp Gly Arg Thr Val Arg Ala Val His Gly Cys His Arg Arg Asp
    50                  55                  60
CCG GTC TGC CGC GAC CTG GTC CGC CAC CCG CGC CTG CTG GGC CCG GCG 240
Pro Val Cys Arg Asp Leu Val Arg His Pro Arg Leu Leu Gly Pro Ala
65                  70                  75                  80
ATG CAG ATC CTG TCC GGC GAC GTG TAC GTC CAC CAG TTC AAG ATC AAC 288
Met Gln Ile Leu Ser Gly Asp Val Tyr Val His Gln Phe Lys Ile Asn
                85                  90                  95
GCG AAG GCC CCG ATG ACC GGC GAT GTC TGG CCG TGG CAC CAG GAC TAC 336
Ala Lys Ala Pro Met Thr Gly Asp Val Trp Pro Trp His Gln Asp Tyr
            100                 105                 110
ATC TTC TGG GCC CGA GAG GAC GGC ATG GAC CGT CCG CAC GTG GTC AAC 384
Ile Phe Trp Ala Arg Glu Asp Gly Met Asp Arg Pro His Val Val Asn
        115                 120                 125
GTC GCG GTC CTG CTC GAC GAG GCC ACC CAC CTC AAC GGG CCG CTG TTG 432
Val Ala Val Leu Leu Asp Glu Ala Thr His Leu Asn Gly Pro Leu Leu
    130                 135                 140
TTC GTG CCG GGC ACC CAC GAG CTG GGC CTC ATC GAC GTG GAG CGC CGC 480
Phe Val Pro Gly Thr His Glu Leu Gly Leu Ile Asp Val Glu Arg Arg
145                 150                 155                 160
GCG CCG GCC GGC GAC GGC GAC GCG CAG TGG CTG CCG CAG CTC AGC GCC 528
Ala Pro Ala Gly Asp Gly Asp Ala Gln Trp Leu Pro Gln Leu Ser Ala
                165                 170                 175
GAC CTC GAC TAC GCC ATC GAC GCC GAC CTG CTG GCC CGG CTG ACG GCC 576
Asp Leu Asp Tyr Ala Ile Asp Ala Asp Leu Leu Ala Arg Leu Thr Ala
            180                 185                 190
GGG CGG GGC ATC GAG TCG GCC ACC GGC CCG GCG GGC TCG ATC CTG CTG 624
Gly Arg Gly Ile Glu Ser Ala Thr Gly Pro Ala Gly Ser Ile Leu Leu
        195                 200                 205
TTC GAC TCC CGG ATC GTG CAC GGC TCG GGC ACG AAC ATG TCG CCG CAC 672
Phe Asp Ser Arg Ile Val His Gly Ser Gly Thr Asn Met Ser Pro His
    210                 215                 220
CCG CGC GGC GTC GTC CTG GTC ACC TAC AAC CGC ACC GAC AAC GCC CTG 720
Pro Arg Gly Val Val Leu Val Thr Tyr Asn Arg Thr Asp Asn Ala Leu
225                 230                 235                 240
CCG GCG CAG GCC GCT CCG CGC CCG GAG TTC CTG GCC GCC CGC GAC GCC 768
Pro Ala Gln Ala Ala Pro Arg Pro Glu Phe Leu Ala Ala Arg Asp Ala
                245                 250                 255
ACC CCG CTG GTG CCG CTG CCC GCG GGC TTC GCG CTG GCC CAG CCC GTC 816
Thr Pro Leu Val Pro Leu Pro Ala Gly Phe Ala Leu Ala Gln Pro Val
            260                 265                 270

INFORMATION FOR SEQ ID NO: 16:
SEQUENCE CHARACTERISTICS:
LENGTH: 20 base pairs
TYPE: nucleic acid
STRANDEDNESS: single
TOPOLOGY: linear
MOLECULE TYPE: other nucleic acid, synthetic DNA

SEQUENCE DESCRIPTION: SEQ ID NO: 16:

AAGCAGTACC GCGAGGCGGG 20

INFORMATION FOR SEQ ID NO: 17:
SEQUENCE CHARACTERISTICS:
LENGTH: 21 base pairs
TYPE: nucleic acid

STRANDEDNESS: single
TOPOLOGY: linear
MOLECULE TYPE: other nucleic acid, synthetic DNA

SEQUENCE DESCRIPTION: SEQ ID NO: 17:

ATGCTGACCC CGACGGAGCT C 21

INFORMATION FOR SEQ ID NO: 18:
SEQUENCE CHARACTERISTICS:
LENGTH: 299 amino acids
TYPE: amino acid
TOPOLOGY: linear
MOLECULE TYPE: protein
ORIGINAL SOURCE:
ORGANISM: Dactylosporangium sp.
STRAIN: RH1
FEATURE:
NAME/KEY: peptide
location: 35 to 299
IDENTIFICATION METHOD: by similarity with known sequence or to an established consensus

### ORIGINAL SOURCE:

ORGANISM: Escherichia coli
IMMEDIATE SOURCE: pBluescriptIIKS+
FEATURE:
NAME/KEY: peptide
location: 1 to 34
IDENTIFICATION METHOD: by similarity with known sequence or to an established consensus

SEQUENCE DESCRIPTION: SEO ID NO: 18:

Met Thr Met Ile Thr Pro Ser Ala Gln Leu Thr Leu Thr Lys Gly Asn
1               5                   10                  15
Lys Ser Trp Val Pro Gly Pro Pro Ser Arg Ser Thr Val Ser Ile Ser
            20                  25                  30
Leu Ile Lys Gln Tyr Arg Glu Ala Gly Tyr Leu Leu Ile Glu Asp Gly
        35                  40                  45
Leu Gly Pro Arg Glu Val Asp Cys Leu Arg Arg Ala Ala Ala Ala Leu
    50                  55                  60

Tyr Ala Gln Asp Ser Pro Asp Arg Thr Leu Glu Lys Asp Gly Arg Thr
65                  70                  75                  80
Val Arg Ala Val His Gly Cys His Arg Arg Asp Pro Val Cys Arg Asp
                85                  90                  95
Leu Val Arg His Pro Arg Leu Leu Gly Pro Ala Met Gln Ile Leu Ser
            100                 105                 110
Gly Asp Val Tyr Val His Gln Phe Lys Ile Asn Ala Lys Ala Pro Met
        115                 120                 125
Thr Gly Asp Val Trp Pro Trp His Gln Asp Tyr Ile Phe Trp Ala Arg
    130                 135                 140
Glu Asp Gly Met Asp Arg Pro His Val Val Asn Val Ala Val Leu Leu
145                 150                 155                 160
Asp Glu Ala Thr His Leu Asn Gly Pro Leu Leu Phe Val Pro Gly Thr
                165                 170                 175
His Glu Leu Gly Leu Ile Asp Val Glu Arg Arg Ala Pro Ala Gly Asp
            180                 185                 190
Gly Asp Ala Gln Trp Leu Pro Gln Leu Ser Ala Asp Leu Asp Tyr Ala
        195                 200                 205
Ile Asp Ala Asp Leu Leu Ala Arg Leu Thr Ala Gly Arg Gly Ile Glu
    210                 215                 220
Ser Ala Thr Gly Pro Ala Gly Ser Ile Leu Leu Phe Asp Ser Arg Ile
225                 230                 235                 240
Val His Gly Ser Gly Thr Asn Met Ser Pro His Pro Arg Gly Val Val
                245                 250                 255
Leu Val Thr Tyr Asn Arg Thr Asp Asn Ala Leu Pro Ala Gln Ala Ala
            260                 265                 270
Pro Arg Pro Glu Phe Leu Ala Ala Arg Asp Ala Thr Pro Leu Val Pro
        275                 280                 285
Leu Pro Ala Gly Phe Ala Leu Ala Gln Pro Val
    290                 295

INFORMATION FOR SEQ ID NO: 19:
SEQUENCE CHARACTERISTICS:
LENGTH: 659 amino acids
TYPE: amino acid
TOPOLOGY: linear
MOLECULE TYPE: protein
ORIGINAL SOURCE:
ORGANISM: Dactylosporangium sp.
STRAIN: RH1
FEATURE:
NAME/KEY: peptide
location: 389 to 659
IDENTIFICATION METHOD: by experiment
ORIGINAL SOURCE:
ORGANISM: Escherichia coli
IMMEDIATE SOURCE: pMAL-c2
FEATURE:
NAME/KEY: peptide
location: 1 to 387
IDENTIFICATION METHOD: by similarity with known sequence or to an established consensus

SEQUENCE DESCRIPTION: SEQ ID NO: 19:

Met Lys Ile Glu Glu Gly Lys Leu Val Ile Trp Ile Asn Gly Asp Lys
1               5                   10                  15
Gly Tyr Asn Gly Leu Ala Glu Val Gly Lys Lys Phe Glu Lys Asp Thr
            20                  25                  30
Gly Ile Lys Val Thr Val Glu His Pro Asp Lys Leu Glu Glu Lys Phe
        35                  40                  45
Pro Gln Val Ala Ala Thr Gly Asp Gly Pro Asp Ile Ile Phe Trp Ala
    50                  55                  60
His Asp Arg Phe Gly Gly Tyr Ala Gln Ser Gly Leu Leu Ala Glu Ile
65                  70                  75                  80
Thr Pro Asp Lys Ala Phe Gln Asp Lys Leu Tyr Pro Phe Thr Trp Asp
                85                  90                  95
Ala Val Arg Tyr Asn Gly Lys Leu Ile Ala Tyr Pro Ile Ala Val Glu
            100                 105                 110
Ala Leu Ser Leu Ile Tyr Asn Lys Asp Leu Leu Pro Asn Pro Pro Lys
        115                 120                 125
Thr Trp Glu Glu Ile Pro Ala Leu Asp Lys Glu Leu Lys Ala Lys Gly
    130                 135                 140
Lys Ser Ala Leu Met Phe Asn Leu Gln Glu Pro Tyr Phe Thr Trp Pro
145                 150                 155                 160
Leu Ile Ala Ala Asp Gly Gly Tyr Ala Phe Lys Tyr Glu Asn Gly Lys
                165                 170                 175
Tyr Asp Ile Lys Asp Val Gly Val Asp Asn Ala Gly Ala Lys Ala Gly
            180                 185                 190
Leu Thr Phe Leu Val Asp Leu Ile Lys Asn Lys His Met Asn Ala Asp
        195                 200                 205
Thr Asp Tyr Ser Ile Ala Glu Ala Ala Phe Asn Lys Gly Glu Thr Ala
    210                 215                 220
Met Thr Ile Asn Gly Pro Trp Ala Trp Ser Asn Ile Asp Thr Ser Lys
225                 230                 235                 240
Val Asn Tyr Gly Val Thr Val Leu Pro Thr Phe Lys Gly Gln Pro Ser
                245                 250                 255
Lys Pro Phe Val Gly Val Leu Ser Ala Gly Ile Asn Ala Ala Ser Pro
            260                 265                 270
Asn Lys Glu Leu Ala Lys Glu Phe Leu Glu Asn Tyr Leu Leu Thr Asp
        275                 280                 285
Glu Gly Leu Glu Ala Val Asn Lys Asp Lys Pro Leu Gly Ala Val Ala
    290                 295                 300
Leu Lys Ser Tyr Glu Glu Glu Leu Ala Lys Asp Pro Arg Ile Ala Ala
305                 310                 315                 320
Thr Met Glu Asn Ala Gln Lys Gly Glu Ile Met Pro Asn Ile Pro Gln
                325                 330                 335
Met Ser Ala Phe Trp Tyr Ala Val Arg Thr Ala Val Ile Asn Ala Ala
            340                 345                 350
Ser Gly Arg Gln Thr Val Asp Glu Ala Leu Lys Asp Ala Gln Thr Asn
        355                 360                 365
Ser Ser Ser Asn Asn Asn Asn Asn Asn Asn Asn Asn Asn Leu Gly Ile
    370                 375                 380
Glu Gly Arg Met Leu Thr Pro Thr Glu Leu Lys Gln Tyr Arg Glu Ala
385                 390                 395                 400
Gly Tyr Leu Leu Ile Glu Asp Gly Leu Gly Pro Arg Glu Val Asp Cys
                405                 410                 415

Leu Arg Arg Ala Ala Ala Ala Leu Tyr Ala Gln Asp Ser Pro Asp Arg
            420                 425                 430
Thr Leu Glu Lys Asp Gly Arg Thr Val Arg Ala Val His Gly Cys His
        435                 440                 445
Arg Arg Asp Pro Val Cys Arg Asp Leu Val Arg His Pro Arg Leu Leu
    450                 455                 460
Gly Pro Ala Met Gln Ile Leu Ser Gly Asp Val Tyr Val His Gln Phe
465                 470                 475                 480
Lys Ile Asn Ala Lys Ala Pro Met Thr Gly Asp Val Trp Pro Trp His
                485                 490                 495
Gln Asp Tyr Ile Phe Trp Ala Arg Glu Asp Gly Met Asp Arg Pro His
            500                 505                 510
Val Val Asn Val Ala Val Leu Leu Asp Glu Ala Thr His Leu Asn Gly
        515                 520                 525
Pro Leu Leu Phe Val Pro Gly Thr His Glu Leu Gly Leu Ile Asp Val
    530                 535                 540
Glu Arg Arg Ala Pro Ala Gly Asp Gly Asp Ala Gln Trp Leu Pro Gln
545                 550                 555                 560
Leu Ser Ala Asp Leu Asp Tyr Ala Ile Asp Ala Asp Leu Leu Ala Arg
                565                 570                 575
Leu Thr Ala Gly Arg Gly Ile Glu Ser Ala Thr Gly Pro Ala Gly Ser
            580                 585                 590
Ile Leu Leu Phe Asp Ser Arg Ile Val His Gly Ser Gly Thr Asn Met
        595                 600                 605
Ser Pro His Pro Arg Gly Val Val Leu Val Thr Tyr Asn Arg Thr Asp
    610                 615                 620
Asn Ala Leu Pro Ala Gln Ala Ala Pro Arg Pro Glu Phe Leu Ala Ala
625                 630                 635                 640
Arg Asp Ala Thr Pro Leu Val Pro Leu Pro Ala Gly Phe Ala Leu Ala
                645                 650                 655
Gln Pro Val

## Claims

1. An isolated nucleic acid fragment coding for a protein having an amino acid sequence selected from sequence Nos. 1, 18 and 19.

2. An isolated nucleic acid fragment selected from nucleotide sequences of Sequence Nos. 2, 8 and 15.

3. An isolated nucleic acid fragment, which hybridizes with the nucleic acid fragment according to claims 1 or 2, and codes for a protein having an enzymatic activity of hydroxylating the 4-position of L-proline and which acts on free L-proline in the presence of 2-ketoglutaric acid and divalent iron ions to produce trans-4-hydroxy-L-proline.

4. The isolated nucleic acid fragment according to claims 1 to 3, wherein the gene is derived from microorganisms belonging to the genus selected from Dactylosporangium, Amycolatopsis and Streptomyces.

5. The isolated nucleic acid fragment according to claim 4, wherein the microorganisms are selected from Dactylosporangium sp. RH1 (FERM BP-4400), Amycolatopsis sp. RH2 (FERM BP-4581), Streptomyces griseoviridis JCM4250 and Streptomyces daghestanicus JCM4365.

6. An expression vector constructed by inserting into a vector a nucleic acid fragment according to claims 1 to 5.

7. A transformed cell having an expression vector according to claim 6.

8. The transformed cell according to claim 7, which is Escherichia coli SOLR/pRH71.

9. A protein having any one of amino acid sequences of Sequence Nos. 18 and 19.

10. A method for producing an L-proline-4-hydroxylase, which comprises cultivating in a medium transformed cells according to claims 7 or 8, thereby producing and accumulating an L-proline-4-hydroxylase, followed by collecting the L-proline-4-hydroxylase from the resulting culture.

11. The method for producing an L-proline-4-hydroxylase according to claim 10, wherein L-proline is added to the medium.

12. A method for producing trans-4-hydrox-L-proline, which comprises cultivating in a medium transformed cells according to claims 7 or 8, thereby producing and accumulating trans-4-hydroxy-L-proline, followed by collecting the trans-4-hydroxy-L-proline from the resulting culture.

13. The method for producing trans-4-hydroxy-L-proline according to claim 12, wherein the transformed cell has the activity of producing L-proline from the saccharide sources in the medium and accumulating L-proline in the culture.

14. The method for producing trans-4-hydroxy-L-proline according to claim 12, wherein the transformed cell has the ability of producing 2-ketoglutaric acid from the saccharide sources in the medium and accumulating 2-ketoglutaric in the culture.

15. The method for producing trans-4-hydroxy-L-prollne according to claim 12, wherein L-proline is added to the medium.

16. The method for producing trans-4-hydroxy-L-proline according to claim 12, wherein L-proline, 2-ketoglutaric acid and divalent iron ions are added to the medium.

17. A method for producing trans-4-hydroxy-L-proline, which comprises culturing in a medium transformed cells according to claim 7 or 8, then converting L-proline into trans-4-hydroxy-L-proline in the presence of 2-ketoglutaric acid and divalent iron ions in the culture or in an aqueous medium while using the culture, the cells cultivated or a product to be prepared by processing the cells as the enzymatic source, followed by collecting the resulting trans-4-hydroxy-L-proline from the culture or the aqueous medium.

18. The method for producing trans-4-hydroxy-L-proline according to claim 17, wherein the transformed cell has the ability of producing L-proline from the saccharide sources in the medium and accumulating L-proline in the culture.

19. The method for producing trans-4-hydroxy-L-proline according to claim 17, wherein the transformed cell has the ability of producing 2-ketoglutaric acid from the saccharide sources in the medium and accumulating 2-ketoglutaric in the culture.

20. The method for producing trans-4-hydroxy-L-proline according to claim 17, wherein the product to be prepared by processing the cells cultivated is selected from dried cells, lyophilized cells, surfactant-treated cells, enzymatically-treated cells, ultrasonically-treated cells, mechanically-ground .cells, solvent-treated cells, fractionated cell proteins, immobilized cells, immobilized cell products, crude enzymes having the enzymatic activity of hydroxylating the 4-position of L-proline to be extracted from the cells, purified products of the crude enzymes and immobilized enzymes.

## Patentansprüche

1. Ein isoliertes Nukleinsäurefragment, das für ein Protein mit der Aminosäuresequenz ausgewählt aus den Sequenzen Nr. 1, 18 und 19 kodiert:

2. Isoliertes Nukleinsäurefragment, ausgewählt aus den Nukleotidsequenzen der Sequenzen Nr. 2, 8 und 15.

3. Isoliertes Nukleinsäurefragment, das mit dem Nukleinsäurefragment gemäß den Ansprüchen 1 oder 2 hybridisiert und für ein Protein kodiert, das die enzymatische Aktivität zur Hydroxylierung der 4-Position von L-Prolin besitzt, und das in Anwesenheit von 2-Ketoglutarsäure und zweiwertigen Eisenionen L-Prolin zu trans-4-Hydroxy-L-Prolin umsetzt.

4. Nukleinsäurefragment gemäß den Ansprüchen 1 bis 3, wobei das Gen gewonnen wird aus Mikroorganismen, die zu den Gattungen ausgewählt aus *Dactylosporangium, Amycolatopsis* und *Streptomyces* gehören.

5. Isoliertes Nukleinsäurefragment gemäß Anspruch 4, wobei die Mikroorganismen ausgewählt sind aus *Dactylosporangium* sp. RH1 (FERM BP-4400), *Amycolatopsis* sp. RH2 (FERM BP-4581), *Streptomyces griseoviridis* JCM4250 und *Streptomyces daghestanicus* JCM4365.

6. Expressionsvektor, hergestellt durch Insertion eines Nukleinsäurefragments gemäß den Ansprüchen 1 bis 5 in einen Vektor.

7. Transformierte Zelle, enthaltend einen Expressionsvektor gemäß Anspruch 6.

8. Transformierte Zelle gemäß Anspruch 7, die *Escherichia coli* SOLR/pRH71 ist.

9. Protein mit einer der Aminosäuresequenzen der Sequenzen Nr. 18 und 19.

10. Verfahren zur Herstellung einer L-Prolin-4-Hydroxylase, umfassend das Kultivieren transformierter Zellen gemäß den Ansprüchen 7 oder 8 in einem Medium, wodurch eine L-Prolin-4-Hydroxylase hergestellt und angereichert wird, gefolgt von Gewinnen der L-Prolin-4-Hydroxylase aus der resultierenden Kultur

11. Verfahren zur Herstellung von L-Prolin-4-Hydroxylase gemäß Anspruch 10, wobei L-Prolin dem Medium zugesetzt wird.

12. Verfahren zur Herstellung von trans-4-Hydroxy-L-Prolin, umfassend das Kultivieren transformierter Zellen gemäß den Ansprüchen 7 oder 8 in einem Medium, wodurch trans-4-Hydroxy-L-Prolin hergestellt und angereichert wird, gefolgt von Gewinnen des trans-4-Hydroxy-L-Prolins aus der resultierenden Kultur.

13. Verfahren zur Herstellung von trans-4-Hydroxy-L-Prolin gemäß Anspruch 12, wobei die transformierte Zelle die Aktivität zur Herstellung von L-Prolin aus Zuckerquellen in dem Medium besitzt, und Anreichern von L-Prolin in der Kultur.

14. Verfahren zur Herstellung von trans-4-Hydroxy-L-Prolin gemäß Anspruch 12, wobei die transformierte Zelle die Fähigkeit zur Herstellung von 2-Ketoglutarsäure aus Zuckerquellen in dem Medium besitzt, und Anreichern von 2-Ketoglutarsäure in der Kultur.

15. Verfahren zur Herstellung von trans-4-Hydroxy-L-Prolin gemäß Anspruch 12, wobei L-Prolin dem Medium zugesetzt wird.

16. Verfahren zur Herstellung von trans-4-Hydroxy-L-Prolin gemäß Anspruch 12, wobei L-Prolin, 2-Ketoglutarsäure und zweiwertige Eisenionen dem Medium zugesetzt werden.

17. Verfahren zur Herstellung von trans-4-Hydroxy-L-Prolin, umfassend Kultivieren transformierter Zellen gemäß Anspruch 7 oder 8 in einem Medium, dann Umsetzen von L-Prolin zu trans-4-Hydroxy-L-Prolin in Anwesenheit von 2-Ketoglutarsäure und zweiwertigen Eisenionen in der Kultur oder in einem wässrigen Medium, während die Kultur, die kultivierten Zellen oder ein Produkt, das durch Verarbeiten der Zellen hergestellt werden soll, als enzymatische Quelle verwendet wird, gefolgt von Gewinnen des resultierenden trans-4-Hydroxy-L-Prolins aus der Kultur oder dem wässrigen Medium.

18. Verfahren zur Herstellung von trans-4-Hydroxy-L-Prolin gemäß Anspruch 17, wobei die transformierte Zelle die Fähigkeit zur Herstellung von L-Prolin aus Zuckerquellen in dem Medium besitzt, und Anreichern von L-Prolin in der Kultur.

19. Verfahren zur Herstellung von trans-4-Hydroxy-L-Prolin gemäß Anspruch 17, wobei die transformierte Zelle die Fähigkeit zur Herstellung von 2-Ketoglutarsäure aus Zuckerquellen in dem Medium besitzt, und Anreichern von 2-Ketoglutarsäure in der Kultur.

20. Verfahren zur Herstellung von trans-4-Hydroxy-L-Prolin gemäß Anspruch 17, wobei das durch Umsetzen der kultivierten Zellen herzustellende Produkt ausgewählt ist aus getrockneten Zellen, lyophilisierten Zellen, Surfaktanzien-behandelten Zellen, enzymatisch behandelten Zellen, ultraschallbehandelten Zellen, mechanisch zermahlenen Zellen, Lösungsmittel-behandelten Zellen, fraktionierten Zellproteinen, immobilisierten Zellen, immobilisierten Zellprodukten, Rohenzymen mit der enzymatischen Aktivität der Hydroxylierung der 4-Position von L-Prolin, das aus den Zellen extrahiert werden soll, gereinigten Produkten aus den Rohenzymen und immobilisierten Enzymen.

## Revendications

1. Fragment d'acide nucléique isolé codant pour une protéine ayant une séquence d'acides aminés choisie parmi les Séquences Nos. 1, 18 et 19.

2. Fragment d'acide nucléique isolé choisi parmi les séquences nucléotidiques de Séquence Nos. 2,8 et 15.

3. Fragment d'acide nucléique isolé, qui hybride avec le fragment d'acide nucléique selon les revendications 1 ou 2, et code pour une protéine ayant une activité enzymatique d'hydroxylation de la position 4 de la L-proline et qui agit sur la L-proline libre en présence d'acide 2-cétoglutarique et d'ions fer divalents pour produire la trans-4-hydroxy-L-proline.

4. Fragment d'acide nucléique isolé selon les revendications 1 à 3, dans lequel le gène est dérivé de micro-organismes appartenant aux genres choisis parmi Dactylosporangium, Amycolatopsis et Streptomyces.

5. Fragment d'acide nucléique isolé selon la revendication 4, dans lequel les micro-organismes sont choisis parmi Dactylosporangium sp. RH1 (FERM BP-4400), Amycolatopsis sp. RH2 (FERM BP-4581), Streptomyces griseoviridis JCM4250 et Streptomyces daghestanicus JCM4365.

6. Vecteur d'expression construit par insertion dans un vecteur d'un fragment d'acide nucléique selon les revendications 1 à 5.

7. Cellule transformée ayant un vecteur d'expression selon la revendication 6.

8. Cellule transformée selon la revendication 7, qui est Escherichia coli SOLR/pRH71.

9. Protéine ayant l'une quelconque des séquences d'acides aminés de Séquence Nos. 18 et 19.

10. Procédé pour la production d'une L-proline-4-hydroxylase, qui comprend la culture dans un milieu de cellules transformées selon les revendications 7 ou 8, produisant et accumulant ainsi une L-proline-4-hydroxylase, suivie par la collecte de la L-proline-4-hydroxylase à partir de la culture résultante.

11. Procédé pour la production d'une L-proline-4-hydroxylase selon la revendication 10, dans lequel de la L-proline est ajoutée au milieu.

12. Procédé pour la production de trans-4-hydroxy-L-proline, qui comprend la culture dans un milieu de cellules transformées selon les revendications 7 ou 8, produisant et accumulant ainsi la trans-4-hydroxy-L-proline, suivie par la collecte de la trans-4-hydroxy-L-proline à partir de la culture résultante.

13. Procédé pour la production de trans-4-hydroxy-L-proline selon la revendication 12, dans lequel la cellule transformée possède l'activité de production de L-proline à partir des sources de saccharide dans le milieu et d'accumulation de L-proline dans la culture.

14. Procédé pour la production de trans-4-hydroxy-L-proline selon la revendication 12, dans lequel la cellule transformée a l'aptitude à produire de l'acide 2-cétoglutarique à partir des sources de saccharide dans le milieu et à accumuler l'acide 2-cétoglutarique dans la culture.

15. Procédé pour la production de trans-4-hydroxy-L-proline selon la revendication 12, dans lequel de la L-proline est ajoutée au milieu.

16. Procédé pour la production de trans-4-hydroxy-L-proline selon la revendication 12, dans lequel de la L-proline, de l'acide 2-cétoglutarique et des ions fer divalents sont ajoutés au milieu.

17. Procédé pour la production de trans-4-hydroxy-L-proline, qui comprend la culture dans un milieu de cellules transformées selon la revendication 7 ou 8, puis la conversion de la L-proline en trans-4-hydroxy-L-proline en présence d'acide 2-cétoglutarique et d'ions fer divalents dans la culture ou dans un milieu aqueux, tandis qu'on utilise la culture, les cellules cultivées ou un produit à préparer par traitement des cellules en tant que source enzymatique, suivie par la récolte de la trans-4-hydroxy-L-proline résultante à partir de la culture ou du milieu aqueux.

18. Procédé pour la production de trans-4-hydroxy-L-proline selon la revendication 17, dans lequel la cellule transformée a l'aptitude à produire de la L-proline à partir des sources de saccharide dans le milieu et à accumuler de la L-proline dans la culture.

19. Procédé pour la production de trans-4-hydroxy-L-proline selon la revendication 17, dans lequel la cellule transformée a l'aptitude à produire de l'acide 2-cétoglutarique à partir des sources de saccharide dans le milieu et à accumuler de l'acide 2-cétoglutarique dans la culture.

20. Procédé pour la production de trans-4-hydroxy-L-proline selon la revendication 17, dans lequel le produit à préparer par traitement des cellules cultivées est choisi parmi les cellules séchées, les cellules lyophilisées, les cellules traitées avec des agents tensio-actifs, les cellules traitées par voie enzymatique, les cellules traitées aux ultrasons, les cellules broyées mécaniquement, les cellules traitées au solvant, les protéines de cellules fractionnées, les cellules immobilisées, les produits de cellules immobilisées, les enzymes brutes ayant l'aptitude enzymatique à hydroxyler la position 4 de la L-proline à extraire des cellules, les produits purifiés des enzymes brutes et les enzymes immobilisées.
